(19) Europäisches Patentamt
European Patent Office
Office européen des brevets

(11) **EP 3 165 164 A1**

(12) **EUROPEAN PATENT APPLICATION**
published in accordance with Art. 153(4) EPC

(43) Date of publication:
**10.05.2017 Bulletin 2017/19**

(51) Int Cl.:
***A61B 5/08*** *(2006.01)*    ***A61B 7/04*** *(2006.01)*

(21) Application number: **14896282.2**

(86) International application number:
**PCT/JP2014/067537**

(22) Date of filing: **01.07.2014**

(87) International publication number:
**WO 2016/002004 (07.01.2016 Gazette 2016/01)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Pioneer Corporation**
**Tokyo 113-0021 (JP)**

(72) Inventors:
• **KAMETANI, Ryushin**
**Kawasaki-shi**
**Kanagawa 212-0031 (JP)**
• **ISHITOYA, Koichi**
**Kawasaki-shi**
**Kanagawa 212-0031 (JP)**

• **OHKUBO, Hideyuki**
**Kawasaki-shi**
**Kanagawa 212-0031 (JP)**
• **MIURA, Tomohiro**
**Kawasaki-shi**
**Kanagawa 212-0031 (JP)**
• **HASEBE, Tsuyoshi**
**Kawasaki-shi**
**Kanagawa 212-0031 (JP)**

(74) Representative: **Viering, Jentschura & Partner mbB**
**Patent- und Rechtsanwälte**
**Am Brauhaus 8**
**01099 Dresden (DE)**

(54) **RESPIRATORY SOUND ANALYSIS DEVICE, RESPIRATORY SOUND ANALYSIS METHOD, COMPUTER PROGRAM, AND RECORDING MEDIUM**

(57)    A breath sound analyzing apparatus is provided with: a first dividing device configured to divide a spectrum of breath sounds, on the basis of a plurality of reference spectra, which are standards for classifying the breath sounds; a second dividing device configured to divide at least one portion of the spectrum divided by the first dividing device, on the basis of a predetermined time-series characteristic; and an outputting device configured to output information regarding ratio of each of divided spectra included in the breath sounds, on the basis of the divided spectra by said first dividing device and said second dividing device. According to the breath sound analyzing apparatus, a plurality of sound types included in the breath sounds can be preferably divided.

FIG. 1

**Description**

Technical Field

The present invention relates to a breath sound analyzing apparatus and a breath sound analyzing method for analyzing breath sounds including a plurality of sound types, a computer program, and a recording medium.

Background Art

For this type of apparatus, there is known an apparatus configured to distinguish between normal breath sounds and abnormal breath sounds in breath sounds of a living body detected by an electronic stethoscope or the like. For example, in Patent Literature 1, there is proposed a technology/technique in which adventitious sounds are identified on the basis of time / frequency expression of conversion signals of the adventitious sounds. In Patent Literature 2, there is proposed a technology/technique in which sound information on abnormal sounds stored in a database is searched for most similar sound information.

Citation List

Patent Literature

Patent Literature 1: Japanese Patent Application Laid Open No. 2004-531309
Patent Literature 2: WO2010/044442

Summary of Invention

Technical Problem

In the technologies/techniques described in the Patent Literatures 1 and 2 described above, however, the normal sound types and the abnormal sound types cannot be sufficiently separated, which is technically problematic. Specifically, for example, wheezes, rhonchi, fine crackles, coarse crackles and the like, which are the abnormal sound types, cannot be respectively separated.
Problems to be solved by the present invention include the aforementioned technical problem as one example. It is therefore an object of the present invention to provide a breath sound analyzing apparatus and a breath sound analyzing method in which a plurality of sound types included in breath sounds can be preferably divided, a computer program, and a recording medium.

Solution to Problem

The above object of the present invention can be achieved by a breath sound analyzing apparatus comprising: a first dividing device configured to divide a spectrum of breath sounds, on the basis of a plurality of reference spectra, which are standards for classifying the breath sounds; a second dividing device configured to divide at least one portion of the spectrum divided by said first dividing device, on the basis of a predetermined time-series characteristic; and an outputting device configured to output information regarding ratio of each of divided spectra included in the breath sounds, on the basis of the divided spectra by said first dividing device and said second dividing device.
The above object of the present invention can be achieved by a breath sound analyzing method comprising: a first dividing process of dividing a spectrum of breath sounds, on the basis of a plurality of reference spectra, which are standards for classifying the breath sounds; a second dividing process of dividing at least one portion of the spectrum divided by said first dividing device, on the basis of a predetermined time-series characteristic; and an outputting process of outputting information regarding ratio of each of divided spectra included in the breath sounds, on the basis of the divided spectra by said first dividing process and said second dividing process.
The above object of the present invention can be achieved by a computer program for making a computer perform: a first dividing process of dividing a spectrum of breath sounds, on the basis of a plurality of reference spectra, which are standards for classifying the breath sounds; a second dividing process of dividing at least one portion of the spectrum divided by said first dividing device, on the basis of a predetermined time-series characteristic; and an outputting process of outputting information regarding ratio of each of divided spectra included in the breath sounds, on the basis of the divided spectra by said first dividing process and said second dividing process.

The above object of the present invention can be achieved by a recording medium according to an embodiment, the computer program described above is recorded.

Brief Description of Drawings

[FIG. 1] FIG. 1 is a block diagram illustrating an entire configuration of a breath sound analyzing apparatus according to an example.

[FIG. 2] FIG. 2 is a flowchart illustrating operations of the breath sound analyzing apparatus according to the example.

[FIG. 3] FIG. 3 is a spectrogram illustrating a frequency analysis result of breath sounds including fine crackles.

[FIG. 4] FIG. 4 is a spectrogram illustrating a frequency analysis result of breath sounds including wheezes.

[FIG. 5] FIG. 5 is a graph illustrating a spectrum in predetermined timing of the breath sounds including fine crackles.

[FIG. 6] FIG. 6 is a conceptual diagram illustrating a method of approximating the spectrum of the breath sounds including fine crackles.

[FIG. 7] FIG. 7 is a graph illustrating a spectrum in predetermined timing of the breath sounds including wheezes.

[FIG. 8] FIG. 8 is a conceptual diagram illustrating a method of approximating the spectrum of the breath sounds including wheezes.

[FIG. 9] FIG. 9 is a graph illustrating one example of a frequency analyzing method.

[FIG. 10] FIG. 10 is a graph illustrating one example of a frequency analysis result.

[FIG. 11] FIG. 11 is a conceptual diagram illustrating a spectrum peak detection result.

[FIG. 12] FIG. 12 is a graph illustrating a basis of normal vesicular sounds.

[FIG. 13] FIG. 13 is a graph illustrating a basis of fine crackles.

[FIG. 14] FIG. 14 is a graph illustrating a basis of continuous pulmonary adventitious sounds.

[FIG. 15] FIG. 15 is a graph illustrating a basis of white noise.

[FIG. 16] FIG. 16A to FIG. 16D are graph illustrating frequency-shifted bases of continuous pulmonary adventitious sounds.

[FIG. 17] FIG. 17 is a diagram illustrating a relation among a spectrum, each basis, and a coupling coefficient.

[FIG. 18] FIG. 18 is a diagram illustrating one example of an observed spectrum and bases used for approximation.

[FIG. 19] FIG. 19 is diagrams each of which illustrates the coupling coefficient and each basis indicating the spectrum.

[FIG. 20] FIG. 20 is version 1 of a conceptual view illustrating a division method using temporal continuity of peak frequency.

[FIG. 21] FIG. 21 is version 2 of a conceptual view illustrating the division method using the temporal continuity of the peak frequency.

[FIG. 22] FIG. 22 is a graph illustrating a threshold value used for division between wheezes and rhonchi according to a first example.

[FIG. 23] FIG. 23 is a graph illustrating an initial value of a threshold value used for division between wheezes and rhonchi according to a second example.

[FIG. 24] FIG. 24 is version 1 of a graph illustrating a value after adjustment of the threshold value used for the division between wheezes and rhonchi according to the second example.

[FIG. 25] FIG. 25 is version 2 of a graph illustrating the value after adjustment of the threshold value used for the division between wheezes and rhonchi according to the second example.

[FIG. 26] FIG. 26 is a spectrogram illustrating breath sounds including wheezes.

[FIG. 27] FIG. 27 is a graph illustrating peak frequency and peak number of wheezes.

[FIG. 28] FIG. 28 is a spectrogram illustrating breath sounds including rhonchi.

[FIG. 29] FIG. 29 is a graph illustrating peak frequency and peak number of rhonchi.

[FIG. 30] FIG. 30 is version 1 of a conceptual view illustrating a division method using temporal continuity of an amplitude value.

[FIG. 31] FIG. 31 is version 2 of a conceptual view illustrating the division method using the temporal continuity of the amplitude value.

[FIG. 32] FIG. 32 is version 1 of a conceptual view illustrating, in order, spectrograms obtained at respective steps of a division process.

[FIG. 33] FIG. 33 is version 2 of a conceptual view illustrating, in order, the spectrograms obtained at respective steps of the division process.

[FIG. 34] FIG. 34 is version 1 of a plan view illustrating a display example on a display unit.

[FIG. 35] FIG. 35 is version 2 of a plan view illustrating a display example on the display unit.

Description of Embodiments

<1>

A breath sound analyzing apparatus according to an embodiment is provided with: a first dividing device configured to divide a spectrum of breath sounds, on the basis of a plurality of reference spectra, which are standards for classifying the breath sounds; a second dividing device configured to divide at least one portion of the spectrum divided by the first dividing device, on the basis of a predetermined time-series characteristic; and an outputting device configured to output information regarding ratio of each of divided spectra included in the breath sounds, on the basis of the divided spectra by the first dividing device and the second dividing device.

According to the breath sound analyzing apparatus in the embodiment, in operation thereof, firstly, the spectrum of the breath sounds is divided by the first dividing device. On the first dividing device, the spectrum of the breath sounds is divided on the basis of the plurality of reference spectra, which are standards for classifying the breath sounds. The "reference spectra" herein are set in advance in accordance with respective sound types, in order to classify the plurality of sound types included in the breath sounds (e.g. normal breath sounds, continuous pulmonary adventitious sounds, fine crackles, etc.). For example, the reference spectra are set as spectra in shapes unique to the respective sound types.

By using the reference spectra, it is possible to know in what ratio each of the sound types corresponding to the respective reference spectra is included in the spectrum of the breath sounds. In other words, it is possible to divide the spectrum of the breath sounds into the sound types corresponding to the respective reference spectra.

After the division by the first dividing device, the at least one portion of the spectrum divided by the first dividing device is divided by the second dividing device. In other words, the spectrum divided by the first dividing device is further divided, at least partially. On the second diving device, the spectrum divided by the first diving device is divided on the basis of the predetermined time-series characteristic. The "predetermined time-series characteristic" herein is a characteristic for determining a time-series change in each spectrum, and is set in advance, for example, in order to determine time-series continuity of peak frequency and an amplitude value, which are unique to each sound type. A plurality of types of predetermined time-series characteristics may be also set.

By using the predetermined time-series characteristic, it is possible to determine whether or not the spectrum includes a sound type with the predetermined time-series characteristic. As a result, the spectrum can be divided into a spectrum of the sound type with the predetermined time-series characteristic and a spectrum of a sound type without the predetermined time-series characteristic.

After the division by the second dividing device, the information regarding the ratio of each of divided spectra included in the breath sounds is outputted by the outputting device, on the basis of the divided spectra by the first dividing device and the second dividing device. In other words, regarding each of the spectra finally obtained as a result of the division by the first dividing device and the second dividing device, the information regarding the ratio of each of the spectra included in the breath sounds is outputted.

Here, even if the division using the reference spectra (i.e. the division by the first diving device) is only used, the spectrum of the breath sounds can be divided to some extent. In the division using the reference spectra, however, it is hard to divide sound types whose reference spectra are similar to each other, or to divide sound types whose reference spectra are hardly set. Thus, there is a possibility that the spectrum of the breath sounds cannot be sufficiently divided only by the division using the reference spectra.

Particularly in the present invention, however, as described above, the at least one portion of the spectrum divided by using the reference spectra is further divided by the division using the predetermined time-series characteristic (i.e. the division by the second dividing device). This makes it possible to preferably divide even the sound type that cannot be divided only by the division using the reference spectra. Specifically, in the division using the predetermined time-series characteristic, for example, continuous pulmonary adventitious sounds and the other sounds can be divided. Moreover, normal breath sounds and coarse crackles can be also divided.

As explained above, according to the breath sound analyzing apparatus in the embodiment, it is possible to preferably divide the spectra included in the breath sounds and to output the information regarding the ratio of each of the sound types included in the breath sounds.

<2>

In one aspect of the breath sound analyzing apparatus according to the embodiment, the first dividing device is provided with: a frequency information obtaining device configured to obtain information regarding frequency corresponding to a first predetermined characteristic of the spectrum of the breath sounds; a shifting device configured to shift the plurality of reference spectra in accordance with the information regarding the frequency, and to obtain frequency-shifted reference spectra; and a first division processing device configured to divide the spectrum of the breath sounds, on the basis of the frequency-shifted reference spectra.

According to this aspect, in the division by the first dividing device, firstly, the information regarding the frequency corresponding to the first predetermined characteristic of the spectrum of the breath sounds is obtained by the frequency information obtaining device. The "first predetermined characteristic" herein means a characteristic that appears in particular frequency in accordance with the sound types included in the spectrum of the breath sounds, and is, for example, a peak(s) that appears in frequency-analyzed signals, or the like. Moreover, the "information regarding the frequency" is not limited to information directly indicating the frequency, but includes in effect information that can indirectly derive the frequency. On the frequency information obtaining device, for example, frequency analysis by Fast Frouier Transform (FFT) or the like is performed on signals indicating the breath sounds, and information regarding frequency corresponding to a local maximum value (i.e. a peak) of an analysis result is obtained.

If the information regarding the frequency is obtained, the plurality of reference spectra, which are standards for classifying the breath sounds, are shifted by the shifting device in accordance with the information regarding the frequency, and the frequency-shifted reference spectra are obtained. The reference spectra are frequency-shifted, for example, in accordance with a peak position(s) or the like, which is the first predetermined characteristic obtained from the breath sounds, and are set as the frequency-shifted reference spectra.

If the frequency-shifted reference spectra are obtained, the spectrum is divided by the first division processing device on the basis of the frequency-shifted reference spectra. In other words, the spectrum of the breath sounds is divided into the sound types corresponding to the respective plurality of frequency-shifted reference spectra. More specifically, for example, an arithmetic operation is performed on the spectrum of the breath sounds by using the plurality of frequency-shifted reference spectra, which are bases. By this, the ratio of each of the frequency-shifted reference spectra included in the spectrum of the breath sounds is calculated as a coupling coefficient. In the calculation of the ratio of the frequency-shifted reference spectra, non-negative approximation (i.e. approximation in which the coupling coefficient is not negative) may be used. As the non-negative approximation, for example, Non-negative Matrix Factorization (NMF) is exemplified.

As explained above, according to the first dividing device in this aspect, the frequency-shifted reference spectra obtained by the shifting based on the breath sounds, which are a division target, are used. It is thus possible to more preferably divide the spectrum of the breath sounds.

<3>

In another aspect of the breath sound analyzing apparatus according to the embodiment, the second dividing device is provided with: a frequency obtaining device configured to obtain frequency corresponding to a second predetermined characteristic of the at least one portion of the spectrum divided by the first dividing device; and a second division processing device configured to divide the at least one portion of the spectrum divided by the first dividing device, in accordance with whether or not the frequency corresponding to the second predetermined characteristic is continued in a time-series.

According to this aspect, in the division by the second dividing device, firstly, the frequency corresponding to the second predetermined characteristic is obtained by the frequency obtaining device from the at least one portion of the spectrum divided by the first dividing device. The "second predetermined characteristic" herein means a characteristic that appears in particular frequency in accordance with the sound types included in a breath sound component, as in the aforementioned first predetermined characteristic, and is, for example, a peak(s) that appears in frequency-analyzed signals, or the like. The second predetermined characteristic may be the same as or different from the first predetermined characteristic. The frequency corresponding to the second predetermined characteristic is obtained a plurality of times in a row, in order to determine temporal continuity described later. A plurality of frequencies obtained in this manner are temporarily stored in a storing device, such as, for example, a buffer.

After the acquisition of the frequency, the at least one portion of the spectrum divided by the first dividing device is divided by the second division processing device. On the second division processing device, the spectrum is divided in accordance with whether or not the frequency corresponding to the second predetermined characteristic is continued in the time-series. The expression "... is continued in the time-series" herein indicates a state in which continuity can be recognized in a time-series change of the obtained frequency, and can be determined, for example, in accordance with whether or not two frequencies obtained in a temporarily continuous manner are in a predetermined frequency range.

If it can be determined whether or not the frequency corresponding to the second predetermined characteristic is continued in the time-series, it is possible to realize division between a sound type with temporal continuity of the second predetermined characteristic (e.g. continuous pulmonary adventitious sounds) and a sound type without the temporal continuity (e.g. normal breath sounds, coarse crackles, etc.). It is thus possible to preferably divide, on the second dividing device, even the sound type that cannot be divided by the division using the reference spectra on the first dividing device, or the sound type that is hardly divided.

<4>

In the aspect in which the second division processing device is provided, the second dividing device may be provided with a third division processing device configured to further divide a spectrum divided by the second division processing device on condition that the frequency corresponding to the second predetermined characteristic is continued in the time-series, in accordance with a relation between the frequency corresponding to the second predetermined characteristic and a predetermined threshold value.

In this case, out of the spectrum divided by the second division processing device, the spectrum divided on condition that the frequency corresponding to the second predetermined characteristic is continued in the time-series (i.e. a spectrum other than a spectrum divided on condition that the frequency corresponding to the second predetermined characteristic is not continued in the time-series) is further divided by the third division processing device.

On the third division processing device, the division is performed in accordance with the relation between the frequency corresponding to the second predetermined characteristic and the predetermined threshold value. The "threshold value" herein is a threshold value for dividing the spectrum divided on condition that the frequency is continued in the time-series, and is set as a value that allows discrimination between two or more different sound types included in the spectrum divided on condition that the frequency is continued in the time-series. By using the predetermined threshold value, for example, it is possible to divide the spectrum divided on condition that the frequency is continued in the time-series, into a spectrum with a frequency of greater than or equal to the predetermined threshold value and a spectrum with a frequency of less than the predetermined threshold value. More specifically, a spectrum corresponding to continuous pulmonary adventitious sounds can be divided into a spectrum corresponding to wheezes and a spectrum corresponding to rhonchi.

A plurality of predetermined threshold value may be also set. Moreover, the predetermined threshold value may be valuable depending on the obtained frequency.

<5>

In another aspect of the breath sound analyzing apparatus according to the embodiment, the second dividing device is provided with: an amplitude value obtaining device configured to obtain an amplitude value of the at least one portion of the spectrum divided by the first dividing device; and a fourth division processing device configured to divide the at least one portion of the spectrum divided by the first dividing device, in accordance with whether or not the amplitude value is continued in a time-series.

According to this aspect, in the division by the second dividing device, firstly, the amplitude value of the at least one portion of the spectrum divided by the first dividing device is obtained by the amplitude value obtaining device. The amplitude value is obtained a plurality of times in a row, in order to determine temporal continuity described later. A plurality of amplitude values obtained in this manner are temporarily stored in a storing device, such as, for example, a buffer.

After the acquisition of the amplitude value, the at least one portion of the spectrum divided by the first dividing device is divided by the fourth division processing device. On the fourth division processing device, the spectrum is divided in accordance with whether or not the obtained amplitude value is continued in the time-series. The expression "... is continued in the time-series" herein indicates a state in which continuity can be recognized in a time-series change of the obtained amplitude value, and can be determined, for example, in accordance with whether or not two amplitude values obtained in a temporarily continuous manner are in a predetermined frequency range.

If it can be determined whether or not the amplitude value is continued in the time-series, it is possible to realize division between a sound type with temporal continuity of the amplitude value (e.g. normal sound types) and a sound type without the temporal continuity (e.g. coarse crackles, etc.). It is thus possible to preferably divide, on the second dividing device, even the sound type that cannot be divided by the division using the reference spectra on the first dividing device, or the sound type that is hardly divided.

If the aforementioned second division processing device (i.e. the device configured to perform the division in accordance with whether or not the frequency is continued in the time-series) is provided in addition to the fourth division processing device, the second division processing device and the fourth division processing device are typically set to divide respective different portions of the spectrum divided by the first dividing device. The spectrum divided by the second division processing device may be further divided by the fourth division processing device. On the other hand, the spectrum divided by the fourth division processing device may be further divided by the second division processing device.

<6>

A breath sound analyzing method according to an embodiment is provided with: a first dividing process of dividing a spectrum of breath sounds, on the basis of a plurality of reference spectra, which are standards for classifying the breath sounds; a second dividing process of dividing at least one portion of the spectrum divided by the first dividing process, on the basis of predetermined time-series characteristics; and an outputting process of outputting information regarding ratio of each of divided spectra included in the breath sounds, on the basis of the divided spectra by the first dividing process and the second dividing process.

According to the breath sound analyzing method in the embodiment, as in the breath sound analyzing apparatus in the embodiment described above, it is possible to preferably divide the spectra included in the breath sounds and to output the information regarding the ratio of each of the sound types included in the breath sounds.

Even the breath sound analyzing method in the embodiment can also adopt the same various aspects as those of the breath sound analyzing apparatus in the embodiment described above.

<7>

A computer program according to an embodiment is configured to make a computer perform: a first dividing process of dividing a spectrum of breath sounds, on the basis of a plurality of reference spectra, which are standards for classifying the breath sounds; a second dividing process of dividing at least one portion of the spectrum divided by the first dividing process, on the basis of predetermined time-series characteristics; and an outputting process of outputting information regarding ratio of each of divided spectra included in the breath sounds, on the basis of the divided spectra by the first dividing process and the second dividing process.

According to the computer program in the embodiment, it can make a computer to perform the same processes as those in the breath sound analyzing method in the embodiment described above. It is therefore possible to preferably divide the spectra included in the breath sounds and to output the information regarding the ratio of each of the sound types included in the breath sounds.

Even the computer program in the embodiment can also adopt the same various aspects as those of the breath sound analyzing apparatus in the embodiment described above.

<8>

On a recording medium according to an embodiment, the computer program described above is recorded.

According to the recording medium in the embodiment, by performing the aforementioned computer program by the computer, it is possible to preferably divide the spectra included in the breath sounds and to output the information regarding the ratio of each of the sound types included in the breath sounds.

The operation and other advantages of the breath sound analyzing apparatus, the breath sound analyzing method, the computer program, and the recording medium according to the embodiments will be explained in more detail in the following examples.

Examples

Hereinafter, a breath sound analyzing apparatus, a breath sound analyzing method, a computer program, and a recording medium according to examples will be explained in detail.

<Entire Configuration>

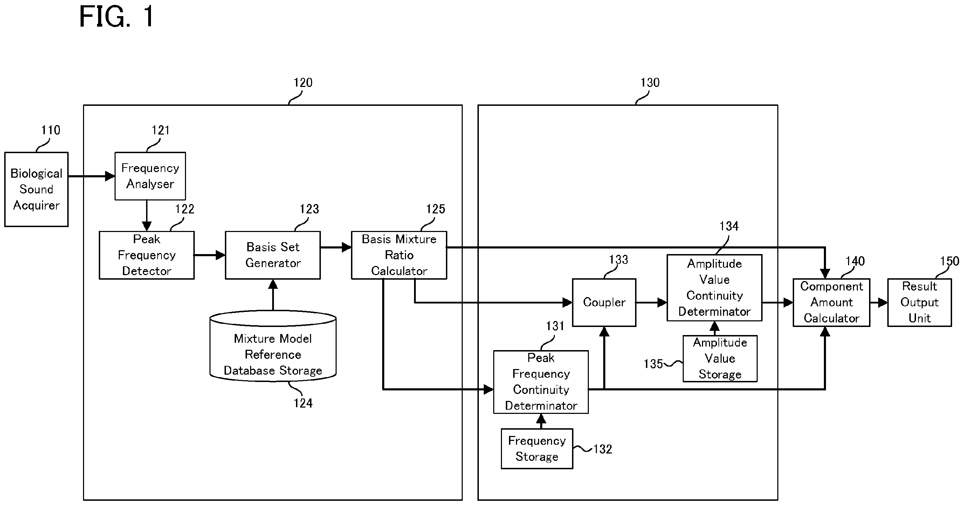

Firstly, an entire configuration of the breath sound analyzing apparatus according to an example will be explained with reference to FIG. 1. FIG. 1 is a block diagram illustrating the entire configuration of the breath sound analyzing apparatus according to the example.

In FIG. 1, the breath sound analyzing apparatus according to the example is provided with a biological sound acquirer 110, a first divider 120, a second divider 130, a component amount calculator 140, and a result output unit 150, as main components.

The biological sound acquirer 110 is configured as a sensor that can obtain breath sounds of a living body, or the like. The biological sound acquirer 110 is provided, for example, with a microphone using an electret condenser microphone (ECM) and a piezoelectric microphone, a vibration sensor, and the like. The breath sounds obtained by the biological sound acquirer 110 are outputted to the first divider 120.

The first divider 120 is one specific example of the "first separating device", and includes a plurality of arithmetic circuits, a memory, and the like. Specifically, the first divider 120 is provided with a frequency analyzer 121, a peak frequency detector 122, a basis set generator 123, a mixture model reference database storage 124, and a basis mixture ratio calculator 125. The first divider 120 is configured to divide the breath sounds obtained by the biological sound acquirer 110 into components corresponding to a plurality of sound types, by using a basis set. A division result of the first divider 120 is outputted to the second divider 130. Operations of parts provided for the first divider 120 will be described in detail later.

The second divider 130 is one specific example of the "second separating device", and includes a plurality of arithmetic circuits, a memory, and the like. Specifically, the second divider 130 is provided with a peak frequency continuity determinator 131, a frequency storage 132, a coupler 133, an amplitude value continuity determinator 134, and an amplitude value storage 135. The second divider 130 is configured to further divide the components corresponding to the plurality of sound types into which the breath sounds are divided by the first divider 120, on the basis of time-series characteristics. A division result of the second divider 130 is outputted to the component amount calculator 140. Operations of parts provided for the second divider 130 will be described in detail later.

The component amount calculator 140 is configured to calculate respective component amounts of the sound types included in the breath sounds obtained by the biological sound acquirer 110, on the basis of the division results of the first divider 120 and the second divider 130. Information indicating the component amounts calculated by the component amount calculator 140 is outputted to the result output unit 150.

The result output unit 150 is configured to output the component amounts calculated by the component amount calculator 140, to a device configured to display images and video, such as, for example, a display, or a device configured to output audio, such as, for example, a speaker.

<Explanation on Operation>

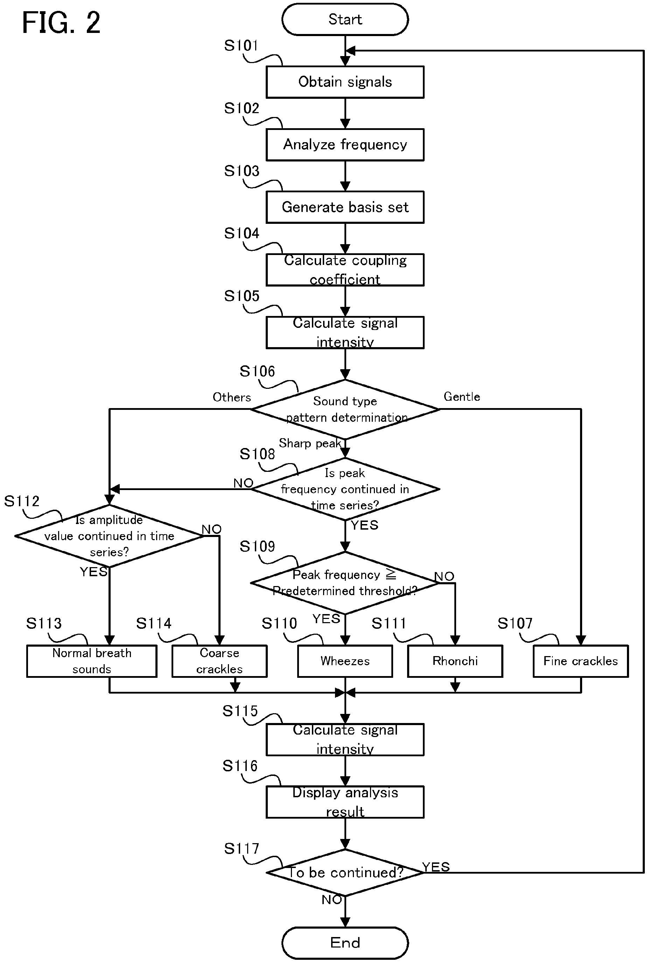

Next, operations of the breath sound analyzing apparatus according to the example will be explained with reference to FIG. 2 in addition to FIG. 1. FIG. 2 is a flowchart illustrating the operations of the breath sound analyzing apparatus according to the example. Here, a simple explanation will be given in order to understand an entire flow of processes of performing the breath sound analyzing apparatus according to the example. The details of each process will be described later.

In FIG. 1 and FIG. 2, in operation of the breath sound analyzing apparatus according to the example, firstly, breath sounds are obtained on the biological sound acquirer 110 (step S101).

If the breath sound signals are obtained, frequency analysis (e.g. Fast Fourier Transform) is performed on the frequency analyzer 121 (step S102). Moreover, a peak(s) (or a local maximum value) is detected on the peak frequency detector 122.

Then, a basis set is generated on the basis set generator 123 (step S103). Specifically, the basis set generator 123 generates the basis set by using bases stored in the mixture model reference database storage 124. At this time, the basis set generator 123 shifts the bases on the basis of positions of the peaks (i.e. corresponding frequency) obtained from a frequency analysis result.

If the basis set is generated, a coupling coefficient (i.e. a value corresponding to the component amount of a sound type corresponding to each basis) is calculated on the basis mixture ratio calculator 125 on the basis of the frequency analysis result and the basis set (step S104), and signal intensity according to the coupling coefficient is calculated (step S105). On the basis mixture ratio calculator 125, sound type pattern determination is further performed on the basis of a calculation result, and divided components are outputted to different portions for respective sound types (step S106).

Specifically, a component characterized by a spectrum in a relatively gentle shape is determined to be a fine crackle component, and is outputted to the component amount calculator 140 (step S107). In other words, the component determined to be fine crackles is not divided on the second divider 130.

Moreover, a component characterized by a spectrum with a sharp peak is determined to be a component mainly including continuous pulmonary adventitious sounds, and is outputted to the peak frequency continuity determinator 131. On the peak frequency continuity determinator 131, it is determined whether or not peak frequency is continued in a time series (step S108). The peak frequency is obtained at predetermined time intervals, and is stored in the frequency storage 132. The peak frequency continuity determinator 131 determines time-series continuity by using the past peak frequencies stored in the frequency storage 132.

Here, regarding components in which it is determined that the peak frequency is continued in the time-series (the step S108: YES), it is further determined whether or not the frequency is greater than or equal to a predetermined threshold value, as a continuous pulmonary adventitious sound component (step S109). A component in which the peak frequency is greater than or equal to the predetermined threshold value (the step S109: YES) is determined to be a wheeze component, and is outputted to the component amount calculator 140 (step S110). On the other hand, a component in which the peak frequency is less than the predetermined threshold value (the step S109: NO) is determined to be a rhonchi component, and is outputted to the component amount calculator 140 (step S111). A component in which it is determined that the peak frequency is not continued in the time-series (the step S108: NO) is outputted to the coupler 133, as a component including normal breath sounds and coarse crackles.

A component determined to exclude the fine crackle component and to exclude the component including continuous pulmonary adventitious sounds is outputted to the coupler 133, as the component including normal breath sounds and coarse crackles. On the coupler 133, the component including normal breath sounds and coarse crackles is coupled with the component in which it is determined on the peak frequency continuity determinator 131 that the peak frequency is not continued in the time-series. The coupled component including normal breath sounds and coarse crackles is outputted to the amplitude value continuity determinator 134.

On the amplitude value continuity determinator 134, it is determined whether or not an amplitude value of a spectrum is continued in a time series (step S112). The amplitude value of the spectrum is obtained at predetermined time intervals and is stored in the amplitude value storage 135. The amplitude value continuity determinator 134 determines time-series continuity by using the past amplitude values stored in the amplitude value storage 135.

Here, a component in which it is determined that the amplitude value is continued in the time series (the step S112: YES) is determined to be a normal breath sound component, and is outputted to the component amount calculator 140 (step S113). On the other hand, a component in which it is determined that the amplitude value is not continued in the time series (the step S112: NO) is determined to be coarse crackle sounds, and is outputted to the component amount calculator 140 (step S114).

Then, on the component amount calculator 140, the signal intensity is calculated on the basis of the division results of the first divider 120 and the second divider 130 (step S115). If the signal intensity is calculated, image data or the like indicating the signal intensity (i.e. the component amounts of sound types included in the breath sounds) is generated on the result output unit 150, and is displayed on an external display or the like as an analysis result (step S116).

Then, it is determined whether or not the analysis process is to be continued (step S117). If it is determined that the analysis process is to be continued (the step S117: YES), process operations from the step S101 are performed again. If it is determined that the analysis process is not to be continued (the step S117: NO), a series of process operations is ended.

<Specific Examples of Breath Sound Signals>

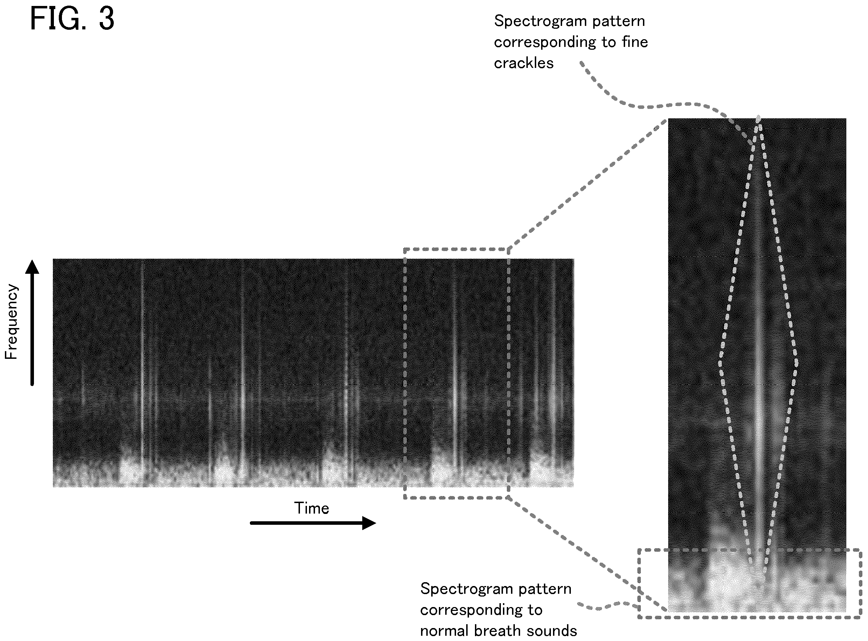

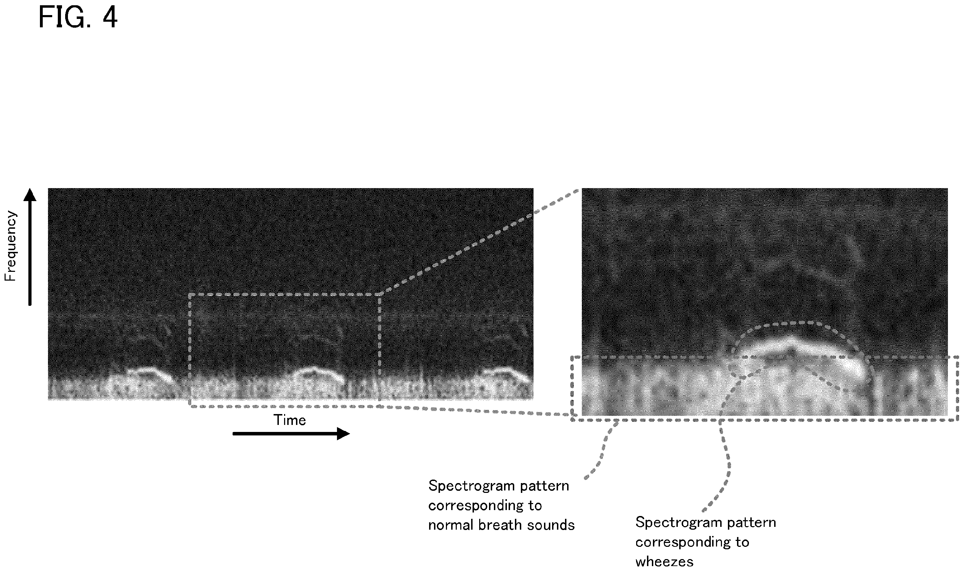

Next, specific examples of the breath sound signals analyzed on the breath sound analyzing apparatus according to the example will be explained with reference to FIG. 3 and FIG. 4. FIG. 3 is a spectrogram illustrating a frequency analysis result of breath sounds including fine crackles. FIG. 4 is a spectrogram illustrating a frequency analysis result of breath sounds including wheezes.

In the example illustrated in FIG. 3, in addition to a spectrogram pattern corresponding to normal breath sounds, a spectrogram pattern corresponding to fine crackles, which is one of abnormal breath sounds, is also observed. The spectrogram pattern corresponding to fine crackle has a shape close to a rhombus, as illustrated in an enlarged part in FIG. 3.

In the example illustrated in FIG. 4, in addition to a spectrogram pattern corresponding to normal breath sounds, a spectrogram pattern corresponding to wheezes, which is one of abnormal breath sounds, is also observed. The spectrogram pattern corresponding to wheezes has a shape close to a swan's neck, as illustrated in an enlarged part in FIG. 4.

As described above, a plurality of sound types exist in abnormal breath sounds, and are observed as spectrogram patterns in different shapes depending on the sound types. As is clear from the drawings, normal breath sounds and abnormal breath sounds are mixedly detected. The breath sound analyzing apparatus according to the example is configured to perform a process for dividing the plurality of sound types which are mixed.

<Method of Approximating Breath Sound Signals>

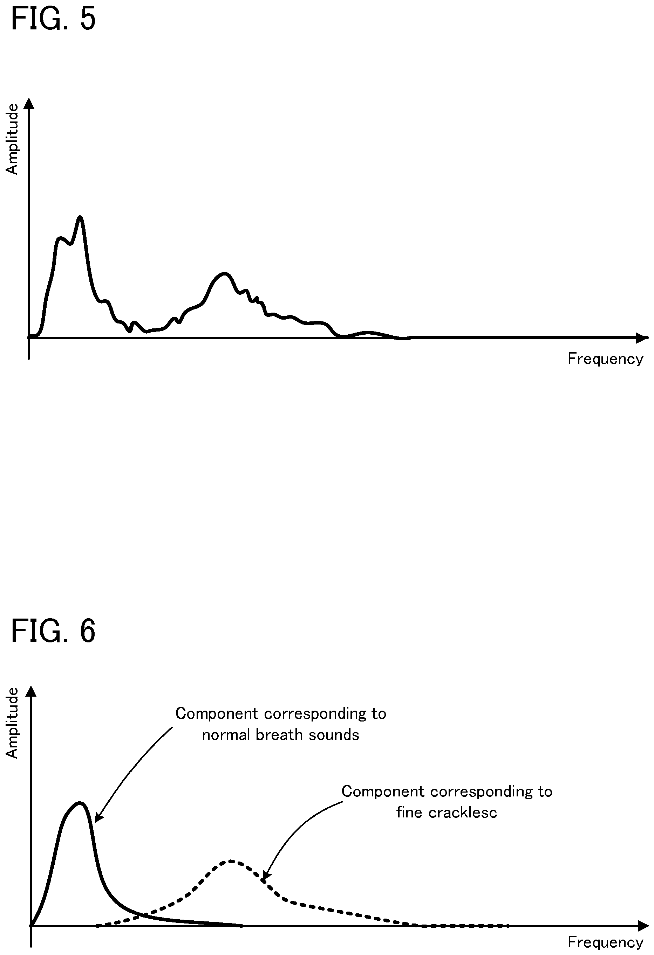

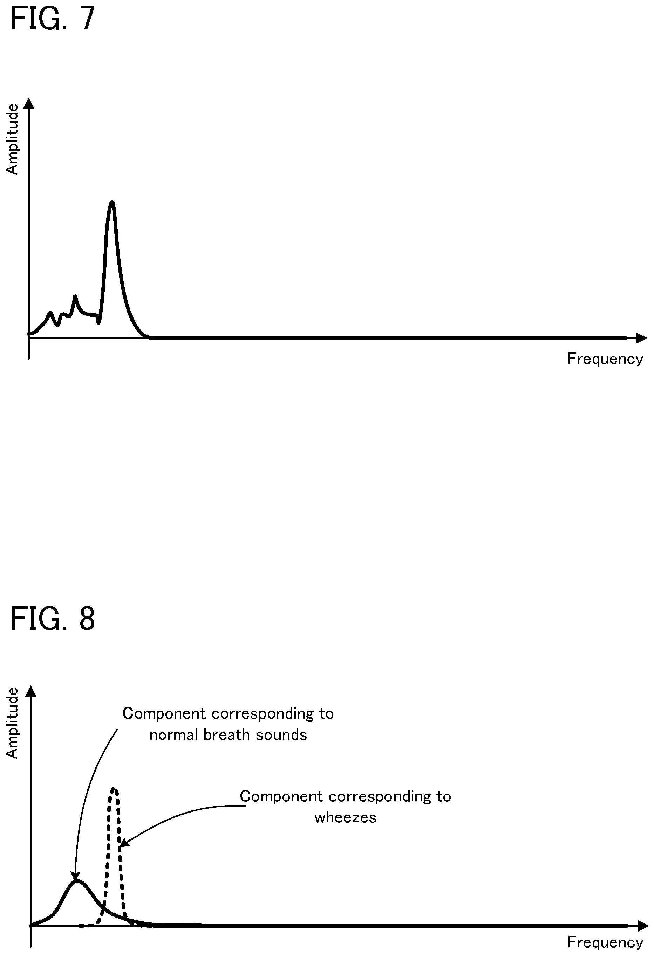

Next, the division process (i.e. division using the basis set) performed by the first divider 120 of the breath sound analyzing apparatus according to the example will be simply explained with reference to FIG. 5 to FIG. 8. FIG. 5 is a graph illustrating a spectrum in predetermined timing of the breath sounds including fine crackles. FIG. 6 is a conceptual diagram illustrating a method of approximating the spectrum of the breath sounds including fine crackles. FIG. 7 is a graph illustrating a spectrum in predetermined timing of the breath sounds including wheezes. FIG. 8 is a conceptual diagram illustrating a method of approximating the spectrum of the breath sounds including wheezes.

In FIG. 5, regarding the breath sound signals including fine crackles (refer to FIG. 3), if a spectrum is extracted in timing of strong appearance of the spectrogram pattern corresponding to fine crackles, a result illustrated in the drawing is obtained. This spectrum is considered to include normal breath sounds and fine crackles.

In FIG. 6, a spectrum corresponding to normal breath sounds and a spectrum corresponding to fine crackles can be estimated in advance by experiments or the like. Thus, by using the patterns estimated in advance, it is possible to know in what rate each of the component corresponding to normal breath sounds and the component corresponding to fine crackles is included with respect to the aforementioned spectrum.

In FIG. 7, regarding the breath sound signals including wheezes (refer to FIG. 4), if a spectrum is extracted in timing of strong appearance of the spectrogram pattern corresponding to wheezes, a result illustrated in the drawing is obtained. This spectrum is considered to include normal breath sounds and wheezes.

In FIG. 8, as in the aforementioned case of normal breath sounds and fine crackles, a spectrum corresponding to wheezes can be also estimated in advance by experiments or the like. Thus, by using the patterns estimated in advance, it is possible to know in what rate each of the component corresponding to normal breath sounds and the component corresponding to wheezes is included with respect to the aforementioned spectrum.

Hereinafter, each process for realizing such analysis will be explained, more specifically.

<Frequency Analysis>

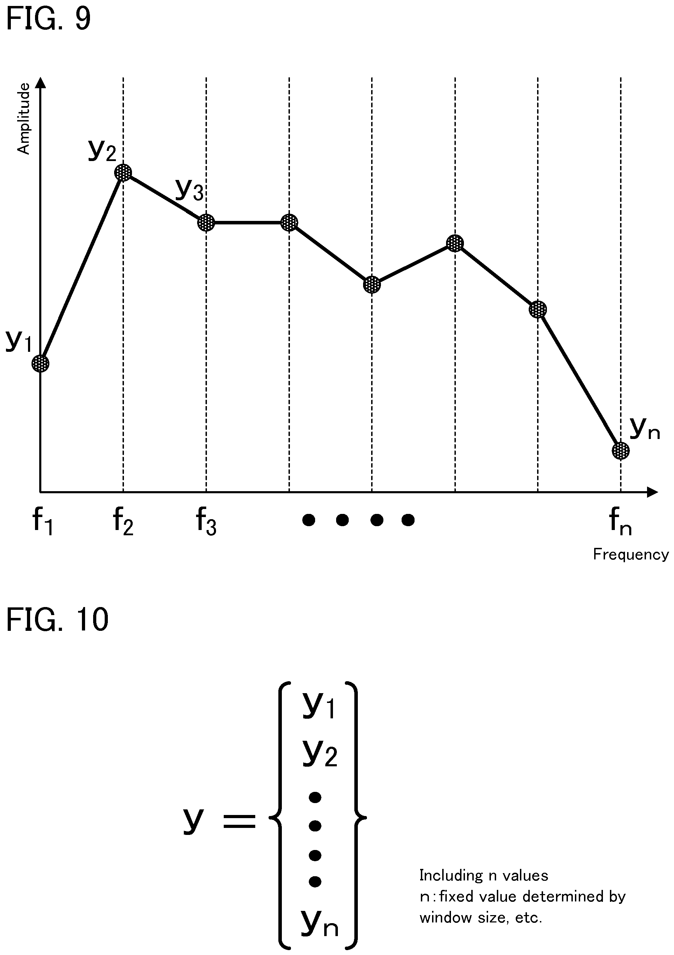

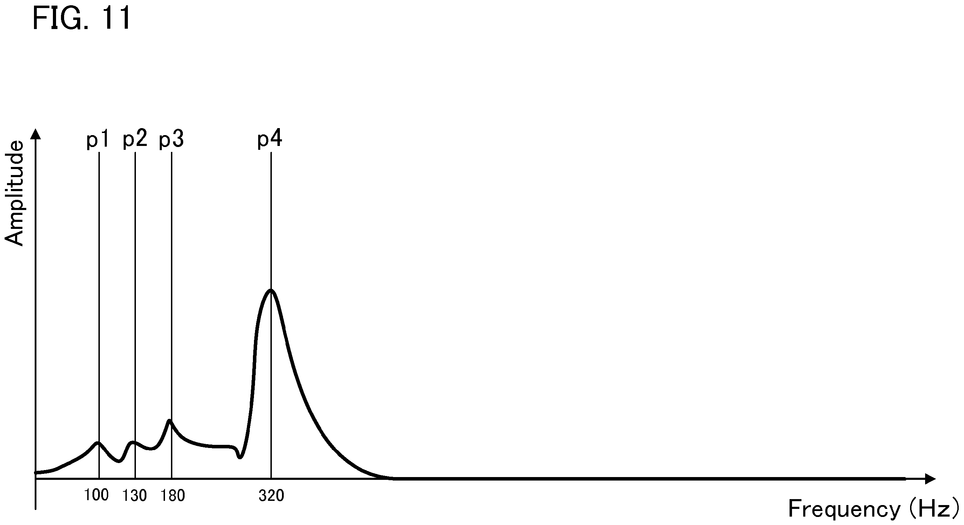

The frequency analysis of breath sound signals and the detection of peaks in the analysis result will be explained in detail with reference to FIG. 9 to FIG. 11. FIG. 9 is a graph illustrating one example of a frequency analyzing method. FIG. 10 is a graph illustrating one example of the frequency analysis result. FIG. 11 is a conceptual diagram illustrating a spectrum peak detection result.

In FIG. 9, firstly, the frequency analysis is performed on the obtained breath sound signals. The frequency analysis can be performed by using the existing technology, such as Fast Fourier Transform. In the example, amplitude values at respective frequencies (i.e. amplitude spectrum) are used as the frequency analysis result. A sampling frequency, a window size, a window function (e.g. a Hanning window, etc.) during data acquisition may be determined, as occasion demands.

As illustrated in FIG. 10, the frequency analysis result includes n values, wherein "n" is a value determined by the window size or the like in the frequency analysis.

In FIG. 11, the peak detection is performed on the spectrum obtained by the frequency analysis. In an example illustrated in FIG. 11, peaks p1 to p4 are respectively detected at positions 100Hz, 130Hz, 180Hz, and 320Hz. The peak detection process may be simple, because it is only necessary to know at which frequency there is a peak. It is, however, preferable to set a parameter for the peak detection so that even a small peak can be detected.

In the example, a point with a local maximum value is obtained, and then, at most N points (wherein N is a predetermined value) are detected in ascending order from a point with the smallest second-order differential value of the obtained point (i.e. in descending order from a point with the largest absolute value). The local maximum value is obtained from a point at which a sign of a difference is changed from positive to negative. The second-order differential value is approximated by a difference of the difference. At most N points with the second-order differential value that is less than a predetermined threshold value, which is negative, are selected from a point with the smallest second-order differential value, and position thereof are stored.

<Generation of Basis Set>

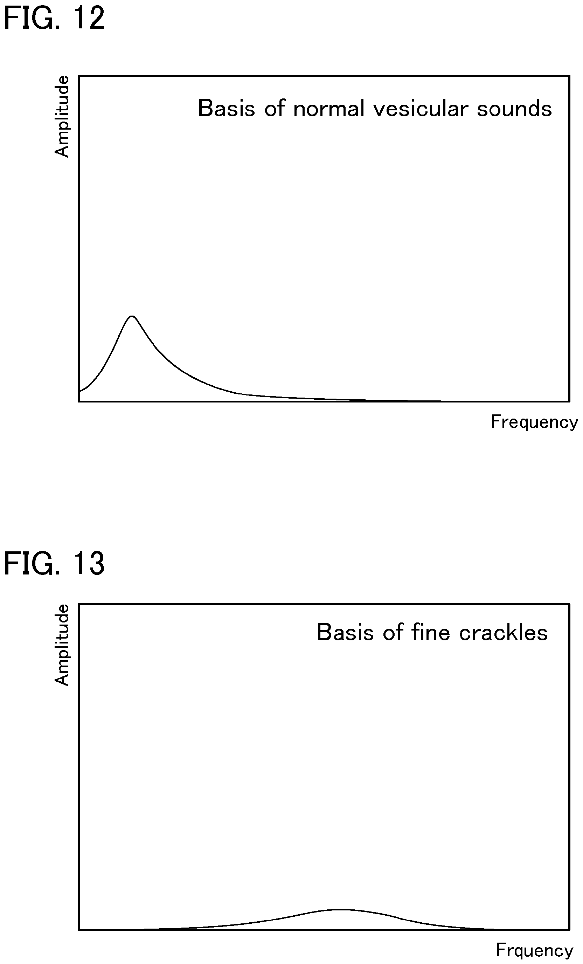

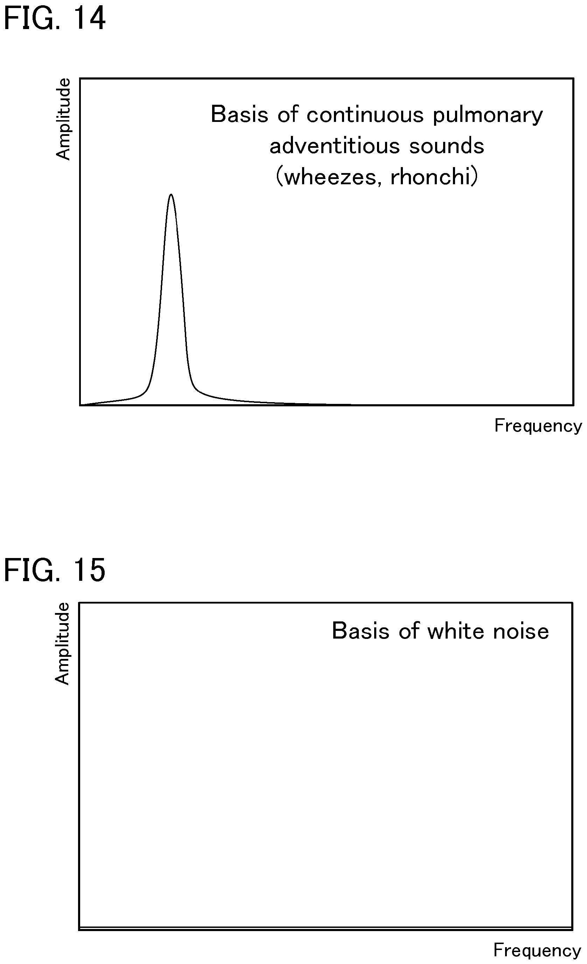

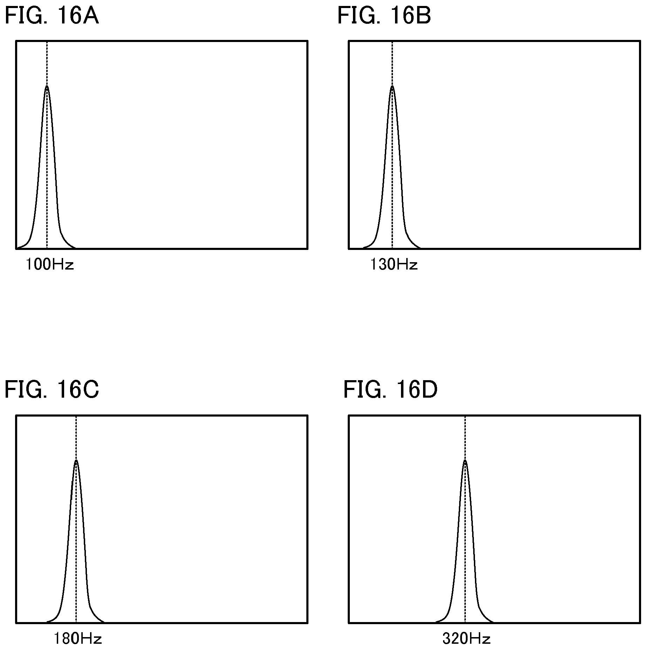

Next, the generation of the basis set will be explained in detail with reference to FIG. 12 to FIG. 16D. FIG. 12 is a graph illustrating a basis of normal vesicular sounds. FIG. 13 is a graph illustrating a basis of fine crackles. FIG. 14 is a graph illustrating a basis of continuous pulmonary adventitious sounds. FIG. 15 is a graph illustrating a basis of white noise. FIG. 16A to FIG. 16D are graphs illustrating frequency-shifted bases of continuous pulmonary adventitious sounds.

As illustrated in FIG. 12 to FIG. 15, each basis corresponding to respective one of the sound types (or one specific example of the "reference spectrum") has a particular shape. Each basis includes n numerical values (i.e. amplitude values at respective frequencies), which are the same as the frequency analysis result. Each basis is normalized so that an area, which is surrounded by a line indicating the amplitude value at each frequency and by a frequency axis, has a predetermined value (e.g. 1).

Here, the four bases, which are the basis of normal vesicular sounds, the basis of fine crackles, the basis of continuous pulmonary adventitious sounds, and the basis of white noise, are illustrated; however, the analysis can be performed even if there is only one basis. Moreover, another basis other than the bases exemplified here can be also used. For example, heartbeat sounds and bowel sounds can be analyzed by using bases corresponding to the heartbeat sounds and the bowel sounds, instead of the bases corresponding to the breath sounds exemplified here.

In FIG. 16A to FIG. 16D, the basis corresponding to continuous pulmonary adventitious sounds out of the aforementioned bases is frequency-shifted in accordance with peak positions detected from the result of the frequency analysis. Here, FIG. 16A to FIG. 16D respectively illustrate examples in which the basis of continuous pulmonary adventitious sounds is frequency-shifted in accordance with the peaks p1 to p4 illustrated in FIG. 11. It is also possible to frequency-shift the bases other than the basis corresponding to continuous pulmonary adventitious sounds.

As a result, the basis set is generated as a set of the basis of normal vesicular sounds, the basis of fine crackles, the bases of continuous pulmonary adventitious sounds, the number of which is the number of the peaks detected, and the basis of white noise.

<Calculation of Coupling Coefficient>

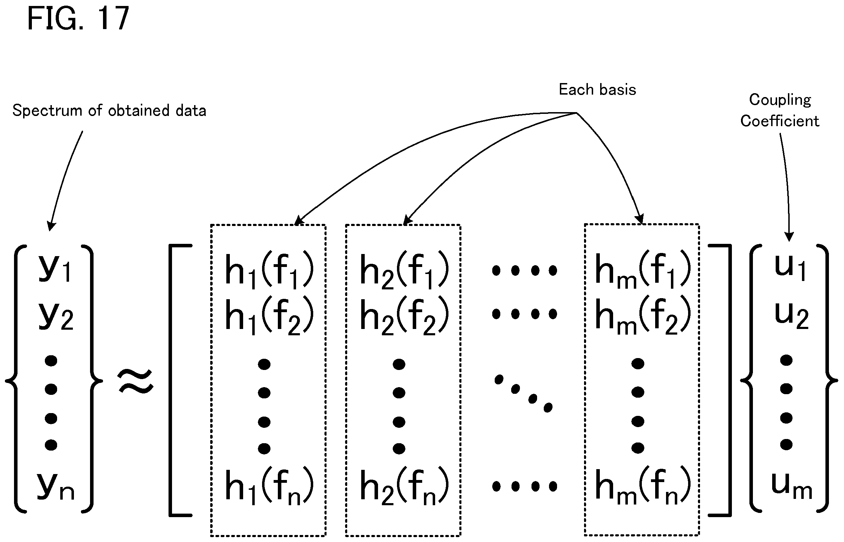

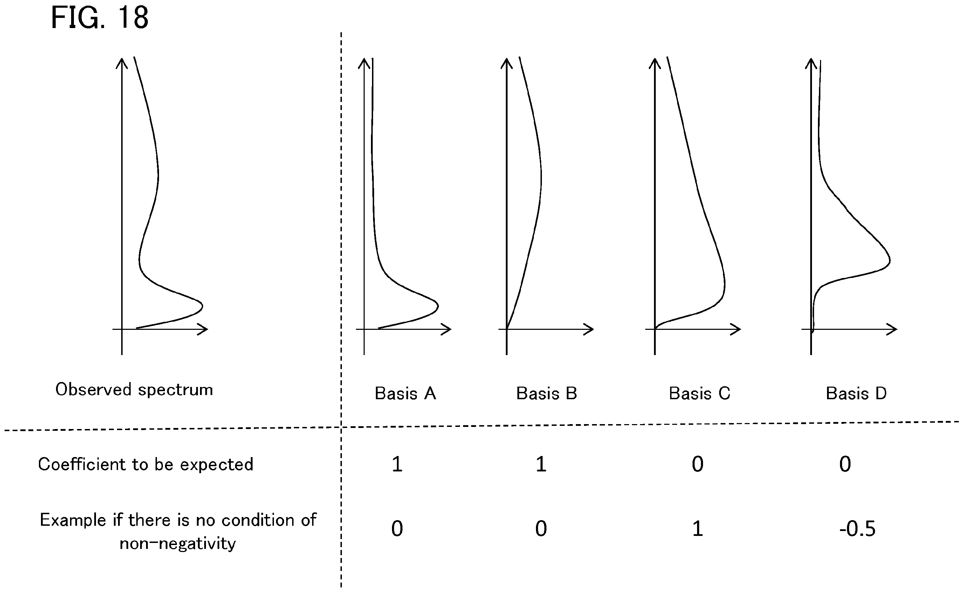

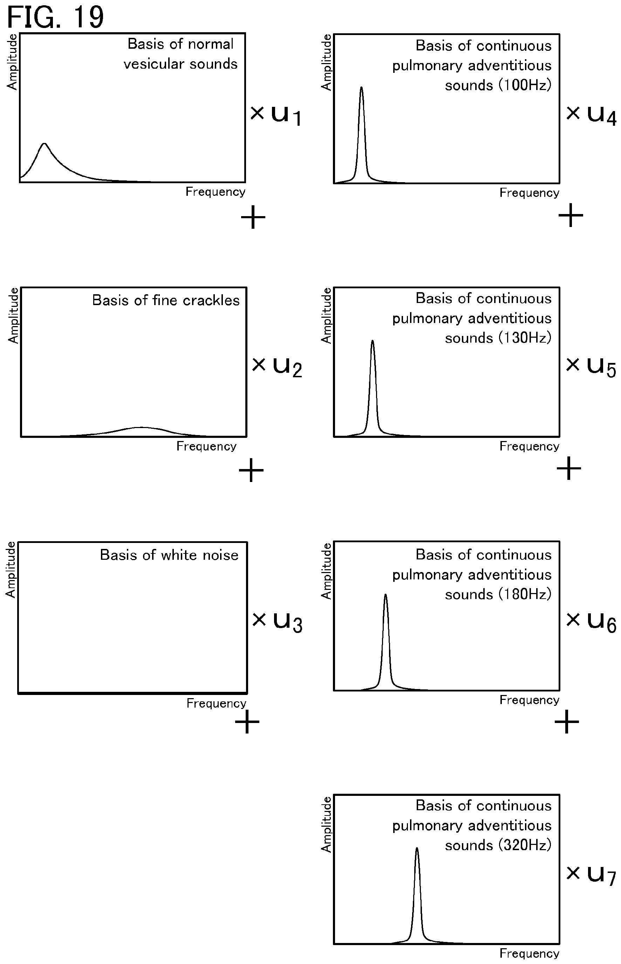

Next, the calculation of the coupling coefficient will be explained in detail with reference to FIG. 17 to FIG. 19. FIG. 17 is a diagram illustrating a relation among the spectrum, each basis, and the coupling coefficient. FIG. 18 is a diagram illustrating one example of an observed spectrum and bases used for approximation. FIG. 19 is diagrams illustrating an approximation result by non-negative matrix factorization

The relation among a spectrum y, a basis h(f), and a coupling coefficient u, which are to be analyzed, can be expressed in the following equation (1).

[Equation 1]

$$y_i \approx \sum_{k=1}^{m} u_k h_k (f_i) \cdots (1)$$

As illustrated in FIG. 17, the spectrum y and each basis h(f) have n values. On the other hand, the coupling coefficient has m values, wherein "m" is the number of the bases included the basis set.

The breath sound analyzing apparatus according to the example is configured to calculate the coupling coefficient of each of the bases included in the basis set by using non-negative matrix factorization. Specifically, it is only necessary to obtain u that minimizes an optimization criterion function D expressed by the following equation (2) (wherein each component value of u is non-negative).

[Equation 2]

$$D = \sum_{i=1}^{n} \left( y_i \log \frac{y_i}{\sum_{k=1}^{m} h_k (f_i) u_k} - y_i + \sum_{k=1}^{m} h_k (f_i) u_k \right) \cdots (2)$$

General non-negative matrix factorization is a method of calculating both a basis matrix, which represents a set of basis spectra, and an activation pattern matrix, which represents the coupling coefficient. In the example, the basis matrix is fixed, and only the coupling coefficient is calculated.

In order to calculate the coupling coefficient, approximation other than the non-negative matrix factorization may be also used. Even in this case, a desired condition is non-negativity. Hereinafter, a reason for the use of the non-negative approximation will be explained with specific examples.

As illustrated in FIG. 18, it is assumed that an observed spectrum is approximated by four bases A to D to calculate the coupling coefficient. If the non-negativity is a condition, the coupling coefficient u to be expected is 1 correspondingly to the basis A, 1 correspondingly to the basis B, 0 correspondingly to the basis C, and 0 correspondingly to the basis D. In other words, if the non-negativity is a condition, the observed spectrum is approximated to a spectrum obtained by adding the basis A multiplied by 1 and the basis B multiplied by 1.

On the other hand, the coupling coefficient u to be expected if the non-negativity is not a condition is 0 correspondingly to the basis A, 0 correspondingly to the basis B, 1 correspondingly to the basis C, and - 0.5 correspondingly to the basis D. In other words, if the non-negativity is not a condition, the observed spectrum is approximated to a spectrum obtained by adding the basis C multiplied by 1 and the basis D multiplied by -0.5.

When the aforementioned two examples are compared, higher approximation accuracy may be obtained if the non-negativity is not a condition, in comparison with a case where the non-negativity is a condition, in some cases. The coupling coefficient u herein, however, represents a component amount of each spectrum, and thus needs to be obtained as a non-negative value. In other words, if the coupling coefficient u is obtained as a negative value, there can be no interpretation as the component amount. In contrast, if the approximation is performed under the non-negativity conditions, the coupling coefficient u corresponding to the component amount can be calculated.

In FIG. 19, the breath sound analyzing apparatus according to the example is configured to calculate the coupling coefficient u by using the basis set including the basis of normal vesicular sounds, the basis of fine crackles, the four bases of continuous pulmonary adventitious sounds, and the basis of white noise, as described above. Thus, the coupling coefficient u is calculated to have seven values $u_1$ to $u_7$.

Here, it may be said that the value $u_1$ corresponding to the basis of normal vesicular sounds is a value indicating ratio of the normal vesicular sounds to the breath sounds. In the same manner, it may be said that each of the value $u_2$

corresponding to the basis of fine crackles, the value $u_3$ corresponding to the basis of white noise, the value $u_4$ corresponding to the basis of continuous pulmonary adventitious sounds shifted to 100Hz, the value $u_5$ corresponding to the basis of continuous pulmonary adventitious sounds shifted to 130Hz, the value $u_6$ corresponding to the basis of continuous pulmonary adventitious sounds shifted to 180Hz, and the value $u_7$ corresponding to the basis of continuous pulmonary adventitious sounds shifted to 320Hz is also a value indicating the ratio of each sound type to the breath sounds. Therefore, the signal intensity of each sound type can be calculated from the coupling coefficient.

As described above, in the example, the plurality of sound types included in the breath sounds are divided by using the plurality of bases corresponding to the respective sound types.

<Division Using Temporal Continuity of Peak Frequency>

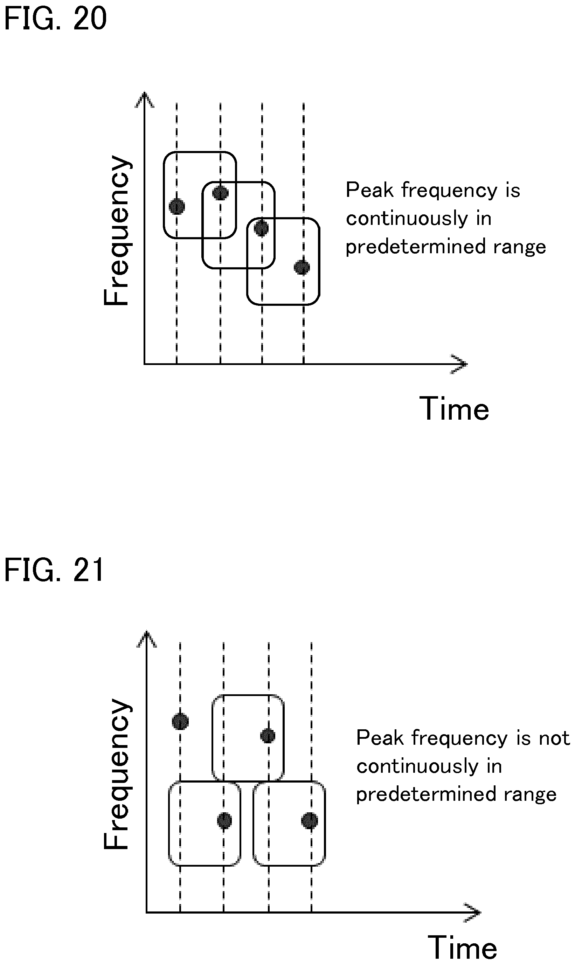

Next, the division process performed on the peak frequency continuity determinator 131 will be specifically explained with reference to FIG. 20 and FIG. 21. Each of FIG. 20 and FIG. 21 is a conceptual view illustrating a division method using temporal continuity of the peak frequency.

The peak frequency continuity determinator 131 is configured to divide one or more components obtained by the division as a component(s) including continuous pulmonary adventitious sounds on the first divider 120, into continuous pulmonary adventitious sounds and other sounds (e.g. normal breath sounds, coarse crackles, etc.), as described above. Specifically, if the peak frequency detected from the frequency analysis result of the breath sound signals varies in a predetermined range, it is determined to be continuous pulmonary adventitious sounds.

As illustrated in FIG. 20, in wheezes and rhonchi, which are continuous pulmonary adventitious sounds, peak positions continuously detected on a time axis vary in the predetermined range. In other words, in wheezes and rhonchi, the peak frequency has temporal continuity. Thus, if the continuous peak positions are in the predetermined range, the sounds can be determined to be continuous pulmonary adventitious sounds.

On the other hand, as illustrated in FIG. 21, in sounds other than the continuous pulmonary adventitious sounds, the peak positions continuously detected on the time axis vary not to be continuously in the predetermined range. In other words, the peak frequency discretely changes without temporal continuity. Thus, if the continuous peak positions are not in the predetermined range, the sounds can be determined to be not the continuous pulmonary adventitious sounds.

For the determination of continuous pulmonary adventitious sounds, a plurality of determination results can be used. Specifically, if the peak positions continuously detected on the time axis vary in the predetermined range a predetermined number of times or more in a row, the sounds may be determined to be continuous pulmonary adventitious sounds.

<Division Using Threshold Value to Peak Frequency>

Next, an explanation will be given to division using the threshold value to the peak frequency, which is performed after the division using the temporal continuity of the peak frequency. Hereinafter, three different examples will be explained.

<First Example>

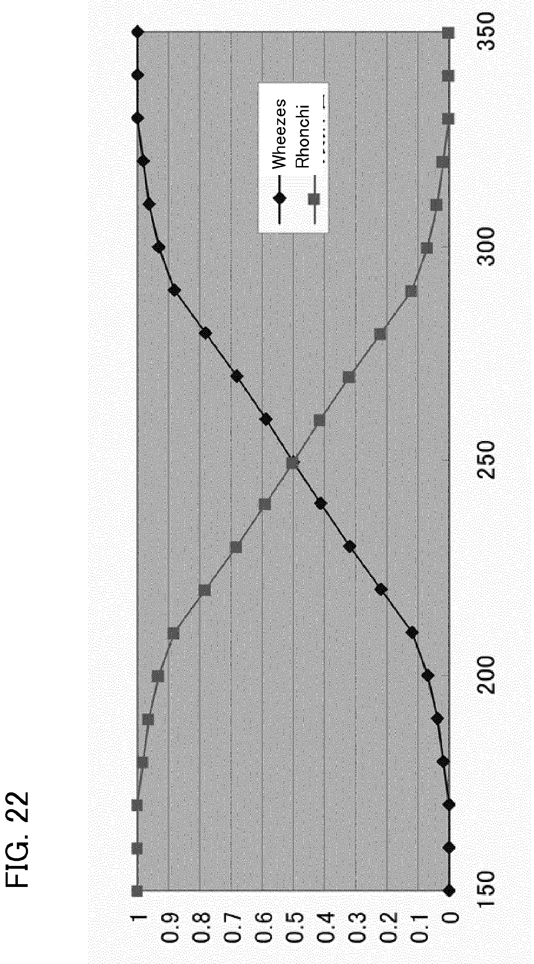

Firstly, a division method using a threshold value according to a first example will be explained with reference to FIG. 22. FIG. 22 is a graph illustrating the threshold value used for division between wheezes and rhonchi according to the first example.

In the division method according to the first example, one or more continuous pulmonary adventitious sound components, which are obtained as a result of the division using the temporal continuity of the peak frequency, are divided into wheezes and rhonchi by being compared with a predetermined threshold value. Here, as is clear from that wheezes are referred to high-pitch continuous sounds and that rhonchi are referred to low-pitch continuous sounds, wheezes and rhonchi can be determined by pitch (i.e. frequency). In wheezes and rhonchi, however, the peak frequency temporarily changes. Thus, if it is desired to use a single threshold value to the peak frequency (i.e. one threshold value that does not vary) in order to determine wheezes and rhonchi, a determination result may change due to a time lapse. For example, if the peak frequency changes across a determination threshold value, what is accurately determined until then will be determined to be a wrong sound type. Thus, in the first example, the determination threshold value is varied depending on the peak frequency.

As illustrated in FIG. 22, in the division process according to the first example, the threshold value varies in such a manner that a ratio at which the sounds are determined to be wheezes and a ratio at which the sounds are determined to be rhonchi smoothly change depending on the peak frequency. For example, in the case of a peak frequency of 200Hz, it is determined that 7% of wheezes and 93% of rhonchi are included. In the case of a peak frequency of 250Hz, it is determined that 50% of wheezes and 50% of rhonchi are included. In the case of a peak frequency of 280Hz, it is determined that 78% of wheezes and 22% of rhonchi are included. The specific numerical values herein are merely one example, and different values may be also set. Moreover, the threshold value may have different variation characteristics depending on sex, age, weight, or the like of a living body which is a measurement target.

The use of the varying threshold value as described above makes it possible to prevent erroneous determination caused by the variation in peak frequency. In other words, in the division process according to the first example, the threshold value for determining wheezes and rhonchi varies to take an appropriate value depending on the peak frequency. Thus, more accurate division can be performed, for example, in comparison with the case of the use of the single threshold value that does not vary.

<Second Example>

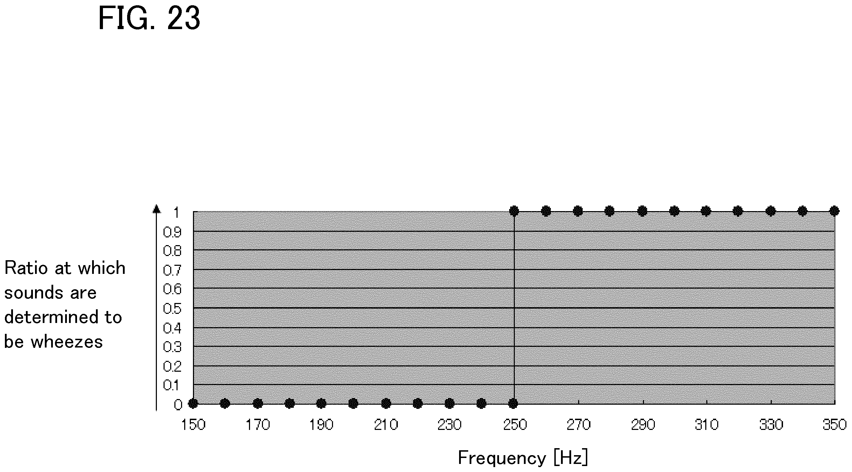

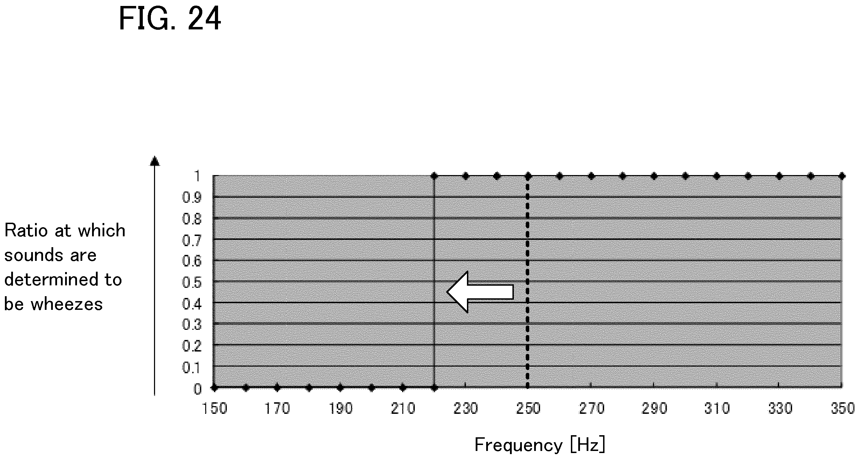

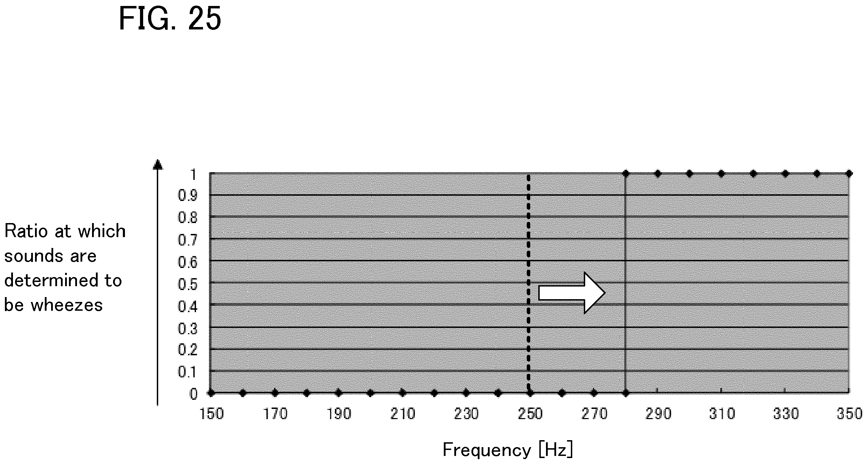

Next, a division process according to a second example will be explained with reference to FIG. 23 to FIG. 25. FIG. 23 is a graph illustrating an initial value of a threshold value used for division between wheezes and rhonchi according to the second example. Each of FIG. 24 and FIG. 25 is a graph illustrating a value after adjustment of the threshold value used for the division between wheezes and rhonchi according to the second example.

As illustrated in FIG. 23, the division process according to the second example is set in such a manner that the determination result changes at a threshold value of 250Hz. Specifically, if the peak frequency is greater than or equal to 250Hz, it is determined that the continuous pulmonary adventitious sounds include 100% of the wheeze component and do not include rhonchi. On the other hand, if the peak frequency is less than 250Hz, it is determined that the continuous pulmonary adventitious sounds include 100% of the rhonchi component and do not include wheezes.

As illustrated in FIG. 24, in the division process according to the second example, if it is determined in the previous determination that 100% of the wheeze component is included, the threshold value is reduced from 250Hz to 220Hz. It is thus easily determined that 100% of the wheeze component is included. Specifically, considering that the peak frequency is 230Hz, the sounds are determined to be rhonchi according to the initial threshold value (refer to FIG. 23), but the sounds are determined to be wheezes according to the threshold value after the adjustment (refer to FIG. 24).

As illustrated in FIG. 25, in the division process according to the second example, if it is determined in the previous determination that 100% of the rhonchi component is included, the threshold value is increased from 250Hz to 280Hz. It is thus easily determined that 100% of the rhonchi component is included. Specifically, considering that the peak frequency is 270Hz, the sounds are determined to be wheezes according to the initial threshold value (refer to FIG. 23), but the sounds are determined to be rhonchi according to the threshold value after the adjustment (refer to FIG. 24).

The adjustment of the threshold value as described above makes it possible to prevent the erroneous determination caused by the variation in peak frequency. In other words, in the division process according to the second example, the threshold value for determining wheezes and rhonchi is adjusted to an appropriate value on the basis of the past determination result. Thus, more accurate determination can be performed, for example, in comparison with the case of the use of the single threshold value that is not adjusted.

The adjustment of the threshold value may be performed not only on the basis of the previous determination result, but also on the basis of a plurality of past determination results. Moreover, if the plurality of past determination results are used, weighting may be performed with respect to each determination result. For example, weighting may be performed to be less influenced in the more distant past determination result. Moreover, as the initial value of the threshold value for the adjustment, the gentle or smooth threshold value in the first example may be used (refer to FIG. 22).

<Third Example>

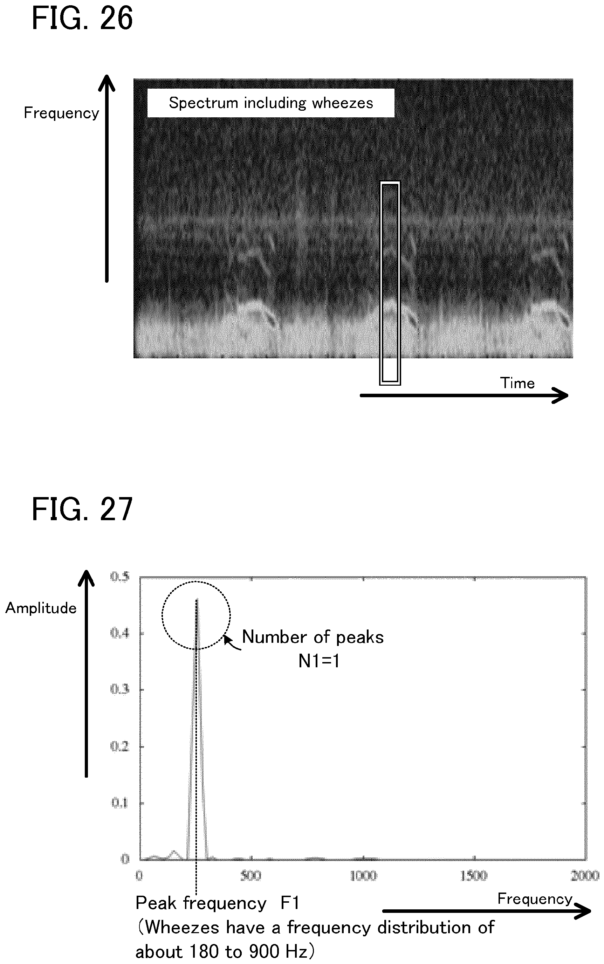

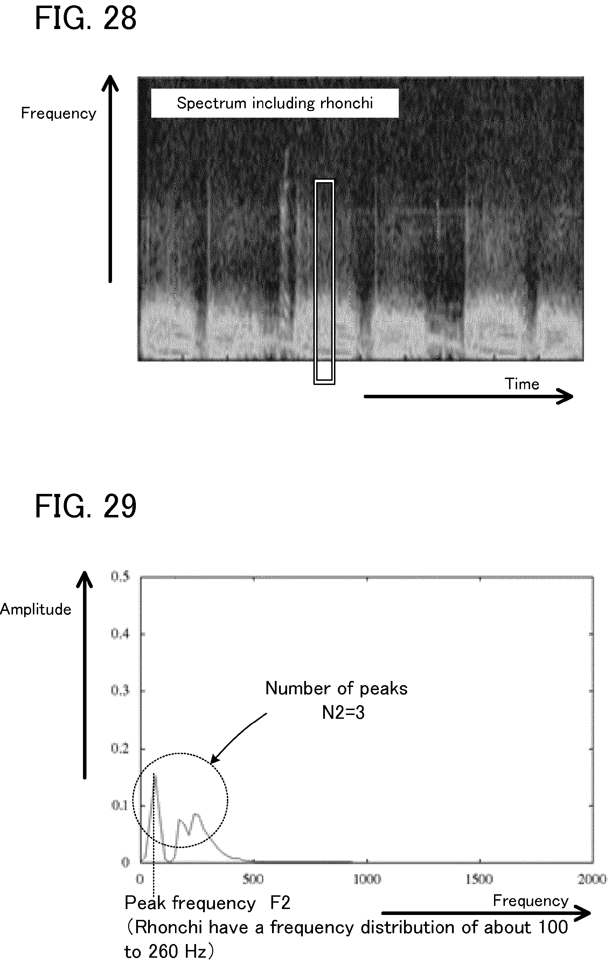

Next, a division process according to a third example will be explained with reference to FIG. 26 to FIG. 29. FIG. 26 is a spectrogram illustrating breath sounds including wheezes. FIG. 27 is a graph illustrating peak frequency and peak number of wheezes. FIG. 28 is a spectrogram illustrating breath sounds including rhonchi. FIG. 29 is a graph illustrating peak frequency and peak number of rhonchi.

In FIG. 26, the breath sounds including wheezes are detected as a spectrum waveform with a predetermined peak. In order to detect peak frequency F and peak number N from there, firstly, a frequency-amplitude graph corresponding to a unit time of the spectrum waveform (i.e. an area surrounded by a white frame in the drawing) is prepared.

From the graph illustrated in FIG. 27, peak frequency F1 and peak number N1 of wheezes can be detected. It is known that wheezes have a peak distribution of about 180 to 900Hz. Moreover, as is clear from the drawing, the peak number N1 of wheezes is 1.

In FIG. 28, the breath sounds including rhonchi are detected as a spectrum waveform with a predetermined peak, which is different from that of wheezes. In order to detect the peak frequency F and the peak number N from here, a frequency-amplitude graph corresponding to a unit time of the spectrum waveform is prepared in the same manner.

From the graph illustrated in FIG. 29, peak frequency F2 and peak number N2 of rhonchi can be detected. It is known that rhonchi have a peak distribution of about 100 to 260Hz. In other words, the peak frequency F2 of rhonchi is distributed in a lower area than that of the peak frequency F1 of wheezes. Moreover, as is clear from the drawing, the peak number N2 of wheezes is not 1 unlike the peak number N1 of wheezes, but is plural number.

In the division process according to the third example, the aforementioned difference in characteristics between wheezes and rhonchi is used for the determination. Specifically, wheezes and rhonchi are divided on the basis of each of the peak frequency F and the peak number N. In this manner, more accurate division can be performed, for example, in comparison with a case where wheezes and rhonchi are divided by using only the peak frequency F.

<Division Using Temporal Continuity of Amplitude Value>

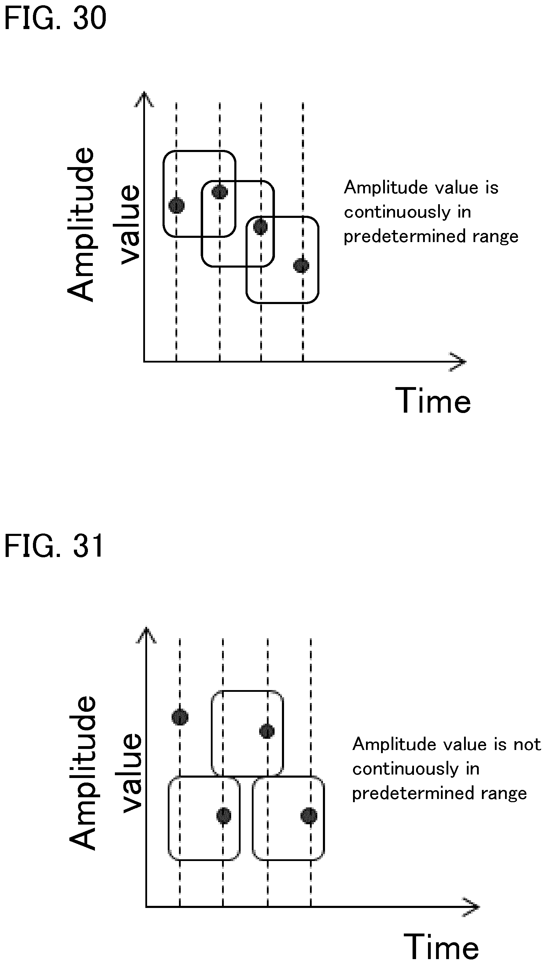

Next, the division process performed on the amplitude value continuity determinator 134 will be specifically explained with reference to FIG. 30 and FIG. 31. Each of FIG. 30 and FIG. 31 is a conceptual view illustrating a division method using temporal continuity of the amplitude value.

The amplitude value continuity determinator 134, as described above, is configured to divide the one or more components obtained by the division as the component(s) including normal breath sounds and coarse crackles on the first divider 120 and the peak frequency continuity determinator 131, into normal breath sounds and coarse crackles. Specifically, the sounds are determined to be normal breath sounds if the amplitude value of the spectrum detected from the frequency analysis result of breath sound signals varies in a predetermined range, and the sounds are determined to be coarse crackles if the amplitude value does not vary in the predetermined range.

As illustrated in FIG. 30, in normal breath sounds, amplitude values continuously detected on a time axis vary in the predetermined range. In other words, the amplitude value changes to have temporal continuity. Thus, the continuous amplitude values are in the predetermined range, the sounds can be determined to be normal breath sounds.

On the other hand, as illustrated in FIG. 31, in coarse crackles, the amplitude values continuously detected on the time axis vary not to be continuously in the predetermined range. In other words, the amplitude value discretely changes without temporal continuity. Thus, the continuous amplitude values are not in the predetermined range, the sounds can be determined to be coarse crackles.

In the division process between normal breath sounds and coarse crackles, a plurality of determination results can be also used. Specifically, if the amplitude values continuously detected on the time axis vary in the predetermined range a predetermined number of times or more in a row, the sounds may be determined to be normal breath sounds, and in the other cases, the sounds may be determined to be coarse crackles.

<Specific Examples of Division Process>

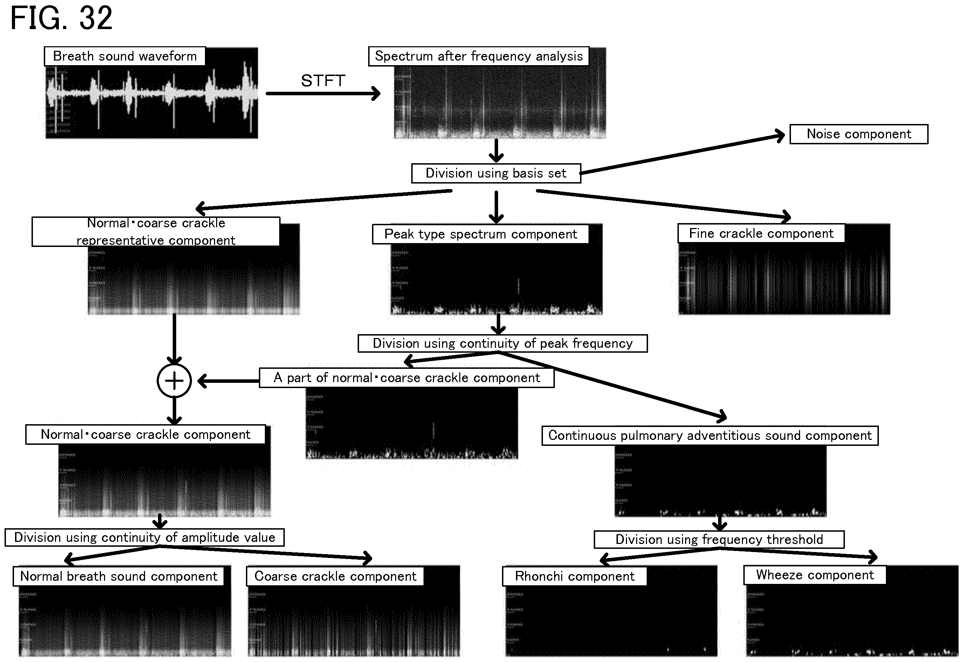

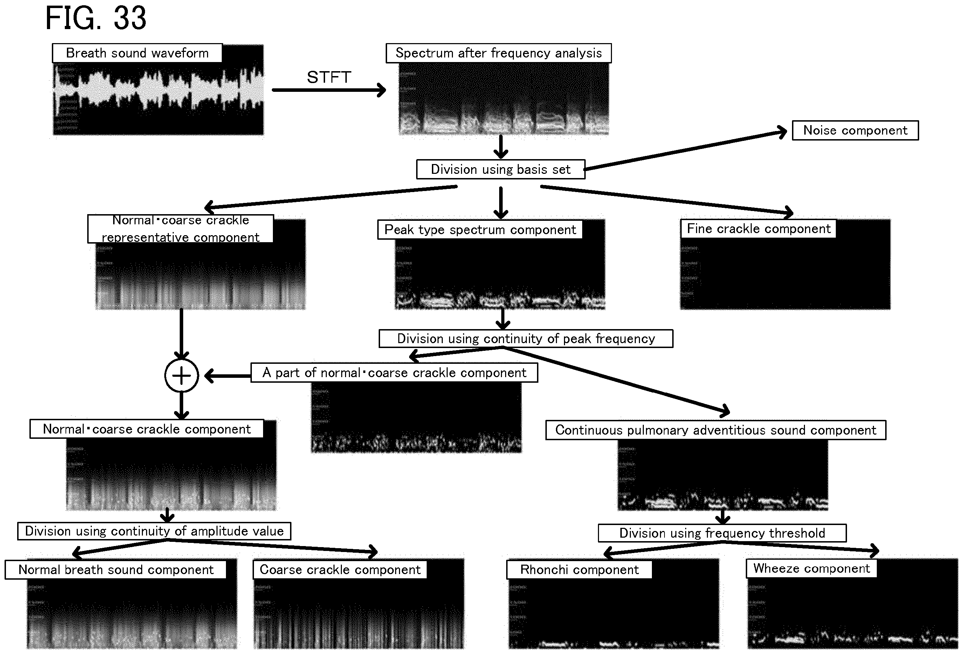

Next, the division process on the breath sound analyzing apparatus according to the example will be more specifically explained with reference to FIG. 32 and FIG. 33. Each of FIG. 32 and FIG. 33 is a conceptual view illustrating, in order, spectrograms obtained at respective steps of the division process.

In an example illustrated in FIG. 32, breath sounds mainly including fine crackles and rarely including wheezes and rchonchi are an analysis target.

Short-time Fourier Transform (STFT) analysis of an obtained breath sound waveform provides a spectrum at intervals of the analysis process. The spectrum after the frequency analysis is divided into a normal / coarse crackle representative component (i.e. the component mainly including normal breath sounds and coarse crackles), a peak type spectrum component (i.e. the component mainly including continuous pulmonary adventitious sounds), the fine crackle component, and a noise component (not illustrated), by the division using the basis set.

The peak type spectrum component is divided into the continuous pulmonary adventitious sound component and a part of the normal / coarse crackle representative component, by the division using the continuity of the peak frequency. The continuous pulmonary adventitious sound component is divided into the rhonchi component and the wheeze component, by the division using the frequency threshold value. On the other hand, a part of the normal / coarse crackle representative component is coupled with the normal / coarse crackle representative component, which is divided by using the basis set, and is then divided into the normal breath sound component and the coarse crackle component, by the division using the continuity of the amplitude value.

Here, as is clear from the spectrogram after the division, the fine crackle component is significantly extracted, while the wheeze component and the rhonchi component are rarely extracted. From this result, it is clear that the sounds types included in the breath sounds can be accurately divided.

On the other hand, in an example illustrated in FIG. 33, breath sounds mainly including wheezes and rhonchi and rarely including fine crackles an analysis target.

In this case, in the spectrogram after the division, the wheeze component and the rhonchi component are significantly extracted, while the fine crackle component is rarely extracted. From this result, as in the example in FIG.

32, it is clear that the sounds types included in the breath sounds can be accurately divided.

<Display Examples of Analysis Result>

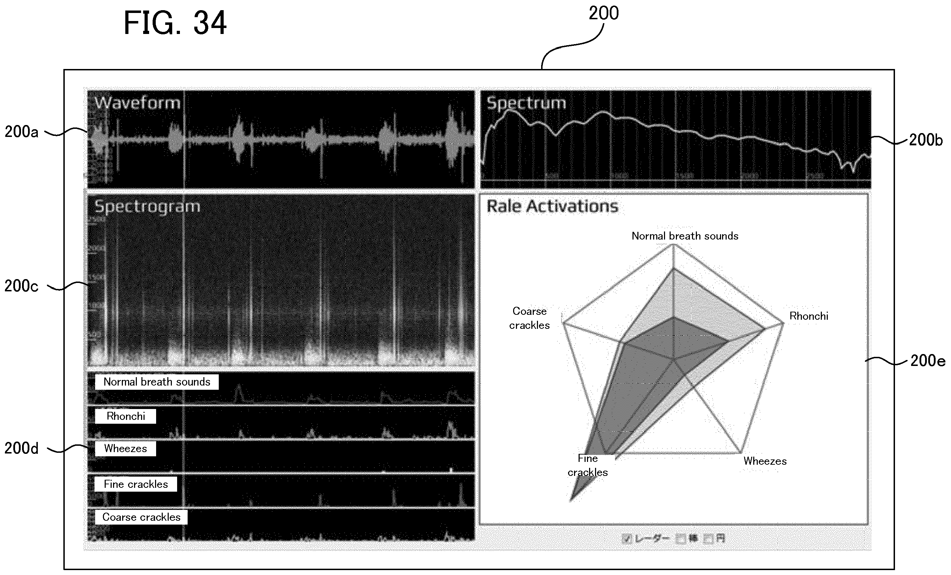

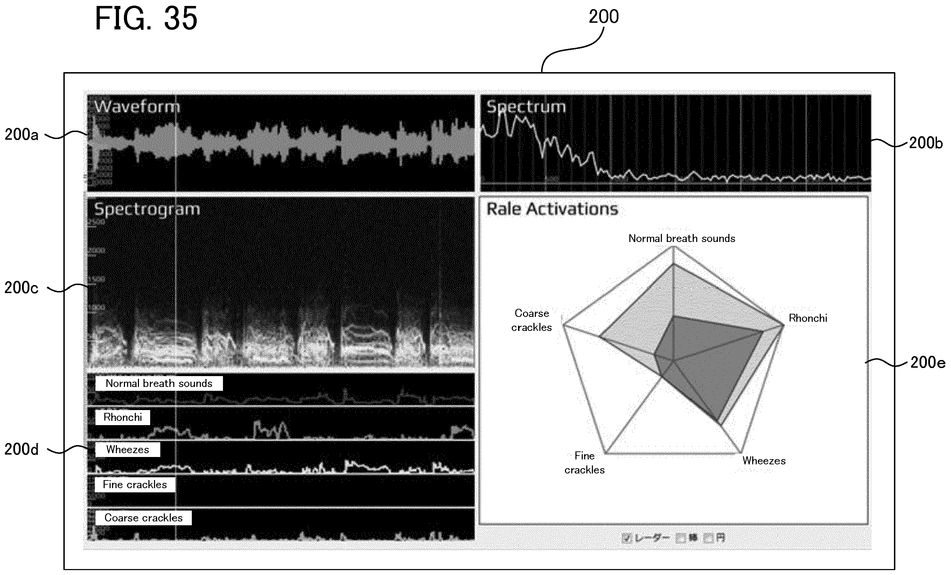

Next, specific display examples of the analysis result will be explained in detail with reference to FIG. 34 and FIG. 35. Each of FIG. 34 and FIG. 35 is a plan view illustrating a display example on a display unit. The display example in FIG. 34 corresponds to the analysis result illustrated in FIG. 32 (i.e. the analysis result of the breath sounds mainly including fine crackles). The display example in FIG. 35 corresponds to the analysis result illustrated in FIG. 33 (i.e. the analysis result of the breath sounds including wheezes and rhonchi).

As illustrated in FIG. 34 and FIG. 35, in a display area 200 of the display unit connected to the breath sound analyzing apparatus according to the example, the analysis result is displayed as a plurality of images. Specifically, in an area 200a, an obtained breath sound waveform is displayed. In an area 200b, an obtained breath sound spectrum is displayed. In an area 200c, an obtained breath sound spectrogram is displayed. In an area 200d, graphs illustrating a time-series change in the component amounts of the divided sound types (which are herein five sound types of normal breath sounds, rhonchi, wheezes, fine crackles, and coarse crackles) are displayed. In an area 200e, ratio of each of the divided sound types is displayed as a radar chart.

The display in this manner makes it possible to provide the analysis result in a visually easy-to-understand manner. In other words, it is possible to intuitively inform a user of the ratio of each of the components included in the breath sounds. Such a display aspect of the analysis result is merely one example, and another display aspect may be used to display the analysis result. For example, the ratio of each of the divided sound types may be displayed as a bar graph or a pie chart, or may be quantified and displayed.

As explained above, according to the breath sound analyzing apparatus in the example, it is possible to preferably divide the spectra included in the breath sounds and to output information regarding the ratio of each of the sound types included in the breath sounds.

The present invention is not limited to the aforementioned embodiments and examples, but various changes may be made, if desired, without departing from the essence or spirit of the invention which can be read from the claims and the entire specification. A breath sound analyzing apparatus, a breath sound analyzing method, a computer program, and a recording medium that involve such changes are also intended to be within the technical scope of the present invention.

Description of Reference Numerals and Letters

| | |
|---|---|
| 110 | breath sound acquirer |
| 120 | first divider |
| 121 | frequency analyzer |
| 122 | peak frequency detector |
| 123 | basis set generator |
| 124 | mixture model reference database storage |
| 125 | basis mixture ratio calculator |
| 130 | second divider |
| 131 | peak frequency continuity determinator |
| 132 | frequency storage |
| 133 | coupler |
| 134 | amplitude value continuity determinator |
| 135 | amplitude value storage |
| 140 | component amount calculator |
| 150 | result output unit |
| 200 | display area |
| y | spectrum |
| h(f) | basis |
| u | coupling coefficient |

**Claims**

1. A breath sound analyzing apparatus comprising:

a first dividing device configured to divide a spectrum of breath sounds, on the basis of a plurality of reference spectra, which are standards for classifying the breath sounds;
a second dividing device configured to divide at least one portion of the spectrum divided by said first dividing device, on the basis of a predetermined time-series characteristic; and
an outputting device configured to output information regarding ratio of each of divided spectra included in the breath sounds, on the basis of the divided spectra by said first dividing device and said second dividing device.

2. The breath sound analyzing apparatus according to claim 1, wherein
said first dividing device comprises:

a frequency information obtaining device configured to obtain information regarding frequency corresponding to a first predetermined characteristic of the spectrum of the breath sounds;
a shifting device configured to shift the plurality of reference spectra in accordance with the information regarding the frequency, and to obtain frequency-shifted reference spectra; and
a first division processing device configured to divide the spectrum of the breath sounds, on the basis of the frequency-shifted reference spectra.

3. The breath sound analyzing apparatus according to claim 1 or 2, wherein said second dividing device comprises:

a frequency obtaining device configured to obtain frequency corresponding to a second predetermined characteristic of the at least one portion of the spectrum divided by said first dividing device; and
a second division processing device configured to divide the at least one portion of the spectrum divided by said first dividing device, in accordance with whether or not the frequency corresponding to the second predetermined characteristic is continued in a time-series.

4. The breath sound analyzing apparatus according to claim 3, wherein said second dividing device comprises a third division processing device configured to further divide a spectrum divided by said second division processing device on condition that the frequency corresponding to the second predetermined characteristic is continued in the time-series, in accordance with a relation between the frequency corresponding to the second predetermined characteristic and a predetermined threshold value.

5. The breath sound analyzing apparatus according to any one of claims 1 to 4, wherein said second dividing device comprises:

an amplitude value obtaining device configured to obtain an amplitude value of the at least one portion of the spectrum divided by said first dividing device; and
a fourth division processing device configured to divide the at least one portion of the spectrum divided by said first dividing device, in accordance with whether or not the amplitude value is continued in a time-series.

6. A breath sound analyzing method comprising:

a first dividing process of dividing a spectrum of breath sounds, on the basis of a plurality of reference spectra, which are standards for classifying the breath sounds;
a second dividing process of dividing at least one portion of the spectrum divided by said first dividing device, on the basis of a predetermined time-series characteristic; and
an outputting process of outputting information regarding ratio of each of divided spectra included in the breath sounds, on the basis of the divided spectra by said first dividing process and said second dividing process.

7. A computer program for making a computer perform:

a first dividing process of dividing a spectrum of breath sounds, on the basis of a plurality of reference spectra, which are standards for classifying the breath sounds;
a second dividing process of dividing at least one portion of the spectrum divided by said first dividing device, on the basis of a predetermined time-series characteristic; and
an outputting process of outputting information regarding ratio of each of divided spectra included in the breath sounds, on the basis of the divided spectra by said first dividing process and said second dividing process.

8. A recording medium on which the computer program according to claim 7 is recorded.

# FIG. 1

110 Biological Sound Acquirer

120
121 Frequency Analyser
122 Peak Frequency Detector
123 Basis Set Generator
124 Mixture Model Reference Database Storage
125 Basis Mixture Ratio Calculator

130
131 Peak Frequency Continuity Determinator
132 Frequency Storage
133 Coupler
134 Amplitude Value Continuity Determinator
135 Amplitude Value Storage

140 Component Amount Calculator

150 Result Output Unit

FIG. 2

```
                        ( Start )
                            │
S101                        ▼
                    ┌─────────────────┐
                    │  Obtain signals │
                    └─────────────────┘
S102                        │
                            ▼
                    ┌─────────────────┐
                    │ Analyze frequency│
                    └─────────────────┘
S103                        │
                            ▼
                    ┌─────────────────┐
                    │ Generate basis set│
                    └─────────────────┘
S104                        │
                            ▼
                    ┌─────────────────┐
                    │ Calculate coupling│
                    │    coefficient   │
                    └─────────────────┘
S105                        │
                            ▼
                    ┌─────────────────┐
                    │ Calculate signal │
                    │    intensity     │
                    └─────────────────┘
S106                        │
         Others             ▼            Gentle
        ◄──────────  ◇ Sound type  ─────────►
                      pattern determination
                         Sharp peak │
S108                        ▼
        NO        ◇ Is peak              ◇
      ◄────────    frequency continued in
                      time series?
                          YES │
S112                          ▼
  ◇ Is amplitude      S109  ◇ Peak frequency ≧   NO
     value continued in      Predetermined threshold? ──►
     time series?  NO
        YES │   ──────►         YES │
S113          S114        S110       S111          S107
 ┌──────────┐ ┌────────┐ ┌────────┐ ┌────────┐ ┌──────────┐
 │ Normal   │ │ Coarse │ │ Wheezes│ │ Rhonchi│ │ Fine     │
 │ breath   │ │crackles│ │        │ │        │ │ crackles │
 │ sounds   │ │        │ │        │ │        │ │          │
 └──────────┘ └────────┘ └────────┘ └────────┘ └──────────┘
S115                        ▼
                    ┌─────────────────┐
                    │ Calculate signal │
                    │    intensity     │
                    └─────────────────┘
S116                        │
                            ▼
                    ┌─────────────────┐
                    │ Display analysis │
                    │     result       │
                    └─────────────────┘
S117                        │
                            ▼            YES
                    ◇ To be continued? ──────►
                          NO │
                            ▼
                        ( End )
```

EP 3 165 164 A1

# FIG. 3

Spectrogram pattern corresponding to fine crackles

Frequency

Time

Spectrogram pattern corresponding to normal breath sounds

FIG. 4

Frequency

Time

Spectrogram pattern corresponding to wheezes

Spectrogram pattern corresponding to normal breath sounds

## FIG. 5

## FIG. 6

FIG. 7

Amplitude

Frequency

FIG. 8

Amplitude

Component corresponding to
normal breath sounds

Component corresponding to
wheezes

Frequency

FIG. 9

FIG. 10

$$y = \left\{ \begin{array}{c} y_1 \\ y_2 \\ \vdots \\ y_n \end{array} \right.$$

Including n values
n : fixed value determined by
window size, etc.

FIG. 11

## FIG. 12

Basis of normal vesicular sounds

Amplitude

Frequency

## FIG. 13

Basis of fine crackles

Amplitude

Frquency

## FIG. 14

Amplitude

Basis of continuous pulmonary
adventitious sounds
(wheezes, rhonchi)

Frequency

## FIG. 15

Amplitude

Basis of white noise

Frequency

## FIG. 16A

100Hz

## FIG. 16B

130Hz

## FIG. 16C

180Hz

## FIG. 16D

320Hz

# FIG. 17

Spectrum of obtained data

Each basis

Coupling Coefficient

$$\left\{\begin{matrix} y_1 \\ y_2 \\ \vdots \\ y_n \end{matrix}\right\} \approx \left[\begin{matrix} h_1(f_1) & h_2(f_1) & \cdots & h_m(f_1) \\ h_1(f_2) & h_2(f_2) & \cdots & h_m(f_2) \\ \vdots & \vdots & & \vdots \\ h_1(f_n) & h_2(f_n) & \cdots & h_m(f_n) \end{matrix}\right] \left\{\begin{matrix} u_1 \\ u_2 \\ \vdots \\ u_m \end{matrix}\right\}$$

EP 3 165 164 A1

# FIG. 18

| Observed spectrum | Basis A | Basis B | Basis C | Basis D |
|---|---|---|---|---|
| Coefficient to be expected | 1 | 1 | 0 | 0 |
| Example if there is no condition of non-negativity | 0 | 0 | 1 | -0.5 |

EP 3 165 164 A1

# FIG. 19

Basis of normal vesicular sounds
Amplitude / Frequency

× u₁

+

Basis of continuous pulmonary adventitious sounds (100Hz)
Amplitude / Frequency

× u₄

+

Basis of fine crackles
Amplitude / Frequency

× u₂

+

Basis of continuous pulmonary adventitious sounds (130Hz)
Amplitude / Frequency

× u₅

+

Basis of white noise
Amplitude / Frequency

× u₃

+

Basis of continuous pulmonary adventitious sounds (180Hz)
Amplitude / Frequency

× u₆

+

Basis of continuous pulmonary adventitious sounds (320Hz)
Amplitude / Frequency

× u₇

## FIG. 20

Peak frequency is
continuously in
predetermined range

Time

## FIG. 21

Peak frequency is not
continuously in
predetermined range

Time

FIG. 22

EP 3 165 164 A1

# FIG. 23

Ratio at which sounds are determined to be wheezes

Frequency [Hz]

EP 3 165 164 A1

FIG. 24

# FIG. 25

## FIG. 26

Frequency

Spectrum including wheezes

Time

## FIG. 27

Amplitude

Number of peaks
N1=1

Peak frequency F1
(Wheezes have a frequency distribution of
about 180 to 900 Hz)

Frequency

# FIG. 28

Frequency

Spectrum including rhonchi

Time

# FIG. 29

Amplitude

Number of peaks
N2=3

Peak frequency  F2
(Rhonchi have a frequency distribution of about 100
to 260 Hz)

Frequency

FIG. 30

Amplitude value is
continuously in
predetermined range

FIG. 31

Amplitude value is not
continuously in
predetermined range

# FIG. 32

Breath sound waveform →STFT→ Spectrum after frequency analysis

→ Noise component

Division using basis set

- Normal·coarse crackle representative component
- Peak type spectrum component
- Fine crackle component

Division using continuity of peak frequency

A part of normal·coarse crackle component → (+)

Continuous pulmonary adventitious sound component

Normal·coarse crackle component

Division using continuity of amplitude value

- Normal breath sound component
- Coarse crackle component

Division using frequency threshold

- Rhonchi component
- Wheeze component

# FIG. 33

Breath sound waveform

— STFT → Spectrum after frequency analysis

→ Noise component

Division using basis set

Normal·coarse crackle representative component

Peak type spectrum component

Fine crackle component

Division using continuity of peak frequency

A part of normal·coarse crackle component → (+)

Continuous pulmonary adventitious sound component

Normal·coarse crackle component

Division using continuity of amplitude value

Normal breath sound component

Coarse crackle component

Division using frequency threshold

Rhonchi component

Wheeze component

EP 3 165 164 A1

FIG. 34

FIG. 35

## INTERNATIONAL SEARCH REPORT

| | International application No. |
|---|---|
| | PCT/JP2014/067537 |

**A. CLASSIFICATION OF SUBJECT MATTER**
*A61B5/08*(2006.01)i, *A61B7/04*(2006.01)i

According to International Patent Classification (IPC) or to both national classification and IPC

**B. FIELDS SEARCHED**

Minimum documentation searched (classification system followed by classification symbols)
A61B5/08, A61B7/04

Documentation searched other than minimum documentation to the extent that such documents are included in the fields searched
Jitsuyo Shinan Koho        1922-1996   Jitsuyo Shinan Toroku Koho   1996-2014
Kokai Jitsuyo Shinan Koho  1971-2014   Toroku Jitsuyo Shinan Koho   1994-2014

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2004-531309 A  (Pneumopartners),<br>14 October 2004 (14.10.2004),<br>paragraphs [0040] to [0050]; fig. 9 to 10<br>& US 2005/0033198 A1     & EP 1381316 A2<br>& WO 2002/082967 A2     & FR 2823660 A1<br>& CA 2445241 A1 | 1-8 |
| A | WO 2010/044452 A1  (Nagasaki University,<br>National University Corp.),<br>22 April 2010 (22.04.2010),<br>paragraphs [0029] to [0054]; fig. 1 to 12<br>& JP 5093537 B2 | 1-8 |
| A | JP 2013-123495 A  (Sharp Corp.),<br>24 June 2013 (24.06.2013),<br>paragraphs [0031] to [0098]; fig. 1 to 12<br>(Family: none) | 1-8 |

☒  Further documents are listed in the continuation of Box C.          ☐  See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered    to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 29 September, 2014 (29.09.14) | 07 October, 2014 (07.10.14) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

| International application No. |
| --- |
| PCT/JP2014/067537 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | WO 1998/14116 A2  (KARMEL MEDICAL ACOUSTIC TECHNOLOGIES LTD.), 09 April 1998 (09.04.1998), claims & JP 2001-505085 A | 1-8 |
| A | JP 2005-66045 A  (Konica Minolta Medical & Graphic, Inc.), 17 March 2005 (17.03.2005), paragraphs [0026] to [0046]; fig. 1 to 2 (Family: none) | 1-8 |

Form PCT/ISA/210 (continuation of second sheet) (July 2009)

**REFERENCES CITED IN THE DESCRIPTION**

**Patent documents cited in the description**

- JP 2004531309 A

- WO 2010044442 A